# EUROPEAN PATENT APPLICATION

(11) **EP 3 715 473 A1**
(43) Date of publication of application: **30.09.2020**
(21) Application number: 19382211.1
(22) Date of filing: 26.03.2019
(51) Int. Cl.: C12Q 1/6883

(54) **PROGNOSTIC MARKERS, THERAPEUTIC TARGET AND TREATMENT FOR ACROMEGALY**

(71) Applicant: Universidade de Santiago de Compostela, 15782 Santiago de Compostela, A Coruña (ES); Servizo Galego de Saúde (SERGAS), 15703 Santiago De Compostela, A Coruña (ES)
(72) Inventor: ÁLVAREZ VILLAMARÍN, Clara, E-15782 Santiago de Compostela, A Coruña (ES); BERNABEU MORÓN, Ignacio, E-15707 Santiago de Compostela, A Coruña (ES)
(74) Representative: ABG Intellectual Property Law, S.L.

(57) **Abstract**

The RET/GDNF survival pathway plays an important role in acromegaly. Hence, ARF gene expression levels are predictive of a good response or resistance to first line therapy with a first-generation analogue of somatostatin. Additionally, Tyrosine Kinase Inhibitor (TKI) able to block the RET/GDNF survival pathway, in particular Sorafenib could be useful for the treatment of acromegaly resistant to first line therapy, by counteracting the survival effect of GDNF or NRTN factors, or the combination of both.

SEQUENCE LISTING

<110>  UNIVERSIDADE DE SANTIAGO DE COMPOSTELA
       SERVIZO GALEGO DE SAÚDE (SERGAS)

<120>  PROGNOSTIC MARKERS, THERAPEUTIC TARGET AND TREATMENT FOR
       ACROMEGALY

<130>  P17567EP00

<160>  22

<170>  PatentIn version 3.5

<210>  1
<211>  18
<212>  DNA
<213>  Artificial Sequence

<220>
<223>  Primer TBP forward

<400>  1
gcccgaaacg ccgaatat                                                    18


<210>  2
<211>  23
<212>  DNA
<213>  Artificial Sequence

<220>
<223>  Primer TBP reverse

<400>  2
ttcgtggctc tcttatcctc atg                                              23


<210>  3
<211>  22
<212>  DNA
<213>  Artificial Sequence

<220>
<223>  Primer TBP Pb 5´FAM, 3¨TAM

<400>  3
tcccaagcgg tttgctgcgg ta                                               22


<210>  4
<211>  22
<212>  DNA
<213>  Artificial Sequence

<220>
<223>  Primer GH1 Syb Forward

<400>  4
gacctagagg aaggcatcca aa                                               22


<210>  5

<211>  23
<212>  DNA
<213>  Artificial Sequence

<220>
<223>  Primer GH1Syb Reverse

<400>  5
agcagcccgt agttcttgag tag                                               23


<210>  6
<211>  22
<212>  DNA
<213>  Artificial Sequence

<220>
<223>  Primer LHB Forward

<400>  6
gcctcctctt cctctaaaga cc                                                22


<210>  7
<211>  21
<212>  DNA
<213>  Artificial Sequence

<220>
<223>  Primer LHB reverse

<400>  7
gcggattgag aagcctttat t                                                 21


<210>  8
<211>  20
<212>  DNA
<213>  Artificial Sequence

<220>
<223>  Primer FSHB forward

<400>  8
ttggtgtgct ggctactgct                                                   20


<210>  9
<211>  20
<212>  DNA
<213>  Artificial Sequence

<220>
<223>  Primer FSHB reverse

<400>  9
gggcactctc actgtttcgt                                                   20


<210>  10
<211>  22
<212>  DNA
<213>  Artificial Sequence

<220>
<223>  Primer LHB forward

<400>  10
gcctcctctt cctctaaaga cc                                                22


<210>  11
<211>  21
<212>  DNA
<213>  Artificial Sequence

<220>
<223>  Primer LHB reverse

<400>  11
gcggattgag aagcctttat t                                                 21


<210>  12
<211>  20
<212>  DNA
<213>  Artificial Sequence

<220>
<223>  Primer FSHB forward

<400>  12
ttggtgtgct ggctactgct                                                   20


<210>  13
<211>  20
<212>  DNA
<213>  Artificial Sequence

<220>
<223>  Primer FSHB reverse

<400>  13
gggcactctc actgtttcgt                                                   20


<210>  14
<211>  21
<212>  DNA
<213>  Artificial Sequence

<220>
<223>  Primer SSTR2 forward

<400>  14
ggcatgtttg actttgtggt g                                                 21


<210>  15
<211>  20
<212>  DNA
<213>  Artificial Sequence

<220>
<223>  Primer SSTR2 reverse

<400>  15
gtctcattca gccgggattt                                                   20


<210>  16
<211>  19
<212>  DNA
<213>  Artificial Sequence

<220>
<223>  Primer SSTR5 forward

<400>  16
ctggtgtttg cgggatgtt                                                    19


<210>  17
<211>  20
<212>  DNA
<213>  Artificial Sequence

<220>
<223>  Primer SSTR5 reverse

<400>  17
gaagctctgg cggaagttgt                                                   20


<210>  18
<211>  24
<212>  DNA
<213>  Artificial Sequence

<220>
<223>  Primer p27 (CDKN1B) Forward

<400>  18
gcaaccgacg attcttctac tcaa                                              24


<210>  19
<211>  21
<212>  DNA
<213>  Artificial Sequence

<220>
<223>  Primer p27(CDKN1B) reverse

<400>  19
cttctgaggc caggcttctt g                                                 21


<210>  20
<211>  24
<212>  DNA
<213>  Artificial Sequence

<220>
<223>  Primer p27(CDKN1B) Pb 5´FAM, 3´TAM

<400>  20
tttcagacgg ttccccaaat gccg                                              24

<210>  21
<211>  20
<212>  DNA
<213>  Artificial Sequence

<220>
<223>  Primer GNAS Forward

<400>  21
ggactctgag ccctctttcc                                                    20


<210>  22
<211>  20
<212>  DNA
<213>  Artificial Sequence

<220>
<223>  Primer GNAS reverse

<400>  22
cacagcatcc taccgttgaa                                                    20

## Description

### FIELD OF THE INVENTION

The invention relates to field of biomarkers for the prognostic of acromegaly. In particular, the invention relates to a method for determining the prognosis of a patient suffering from acromegaly, or for predicting the clinical response of said patient to a first-line treatment of acromegaly, or for selecting an acromegaly patient for a therapy. The invention also relates to the use of a Tyrosine Kinase Inhibitor that blocks the RET/GDNF survival pathway for use in the treatment of acromegaly.

### BACKGROUND OF THE INVENTION

The pituitary gland, or hypophysis, is an endocrine gland about the size of a pea and weighing 0.5 grams in humans. It is a protrusion off the bottom of the hypothalamus at the base of the brain. The pituitary rests upon the hypophysial fossa of the sphenoid bone in the center of the middle cranial fossa and is surrounded by a small bony cavity (sella turcica) covered by a dural fold (diaphragma sellae). Hormones secreted from the pituitary gland help in controlling growth, blood pressure, energy management, all functions of the sex organs, thyroid glands and metabolism as well as some aspects of pregnancy, childbirth, nursing, water/salt concentration at the kidneys, temperature regulation and pain relief.

The anterior pituitary contains several different types of cells that synthesize and secrete hormones. Usually there is one type of cell for each major hormone formed in anterior pituitary. With special stains attached to high-affinity antibodies that bind with distinctive hormone, at least 5 types of cells have been differentiated:
Somatotropes (more than half of secretory cells in normal pituitary): secretes human growth hormone (hGH)
Corticotropes: secrete adrenocorticotropin (ACTH)
Thyrotropes: secrete thyroid stimulating hormone (TSH)
Gonadotropes secrete gonadotropic hormone i.e., both luteinizing hormone (LH) and follicle stimulating hormone (FSH)
Lactotropes secrete prolactin (PRL)

Hormone secretion from the anterior pituitary gland is regulated by hormones secreted by the hypothalamus. Human growth hormone (hGH), also referred to as somatotropin, is released under the influence of hypothalamic growth hormone-releasing hormone (GHRH) or somatocrinin, and is inhibited by hypothalamic somatostatin. Somatotropic cells in the anterior pituitary gland synthesize and secrete hGH in a pulsatile manner, in response to these stimuli by the hypothalamus.

hGH is a peptide hormone, it stimulates growth, cell reproduction, and cell regeneration in humans. hGH also stimulates production of insulin-like growth factor 1 (IGF-1) and raises the concentration of glucose and free fatty acids. It is a type of mitogen which is specific only to the receptors on certain types of cells.

Pituitary tumors represent 16% (11-22%) of intracranial neoplasms. Hyperpituitarism is the condition where the pituitary secretes excessive amounts of hormones. This hypersecretion often results in the formation of a pituitary adenoma (tumour), which are benign apart from a tiny fraction. There are mainly three types of anterior pituitary tumors and their associated disorders. The most common type of pituitary tumour is a prolactinoma which hypersecretes prolactin. A second type of pituitary adenoma secretes excess ACTH, which in turn, causes an excess of cortisol to be secreted and is the cause of Cushing's disease.

Acromegaly, the third type, results from excessive secretion of growth hormone (hGH) often being released by a pituitary adenoma. This disorder can cause disfigurement and possibly death and can lead to gigantism. It is a rare disease caused by a benign pituitary adenoma of somatotroph cells (ACRO). The majority of cases are sporadic, although familial cases have been described. Acromegaly has symptoms derived from expansion of the tumor compressing the pituitary or the surrounding structures such as the cavernous sinus, optic chiasma or hypothalamus. Additionally, excess hGH and consequently elevated IGF-1 levels, produce peripheral signs with growth of soft tissues and multiple comorbidities (metabolic, cardiovascular, oncological, etc.)

First-line therapy of Acromegaly initially comprises endoscopic surgery aiming to cure the disease without damaging the remaining pituitary gland. However, the surgical cure rate is only around 50% (40-65%), being inversely related to tumor size and invasiveness outside the sella turcica.

Patients not cured by surgery undergo adjuvant therapy with first generation somatostatin analogues (fgSSA), such as octreotide and lanreotide. They are considered the mainstay in the medical management of acromegaly, however, only half of patients not cured by surgery respond to fgSSA. In fgSSA-resistant cases second-line therapies include combined treatment with dopamine analogs, pegvisomant or pasireotide, and even re-intervention or radiotherapy. These procedures have a range of side effects without necessarily controlling the apparently benign tumor. Therefore, there is an urgent need for new targets and better treatments of acromegaly.

RET (REarranged during Transfection) is a tyrosine kinase receptor activated by a ligand in the presence of a membrane co-receptor. In mammals there are four different ligands for RET (Glial cell line-Derived Neurotrophic Factor (GDNF), Neurturin (NRTN), Artemin (ARTM), Persephin (PSPN) and four respective co-receptors (GFRA1-4) (Ibanez CF et al.: "Biology of GDNF and its receptors - Relevance for disorders of the central nervous system"; Neurobiol Dis 2017; 97:80-89). In addition to the respective ligand-co-receptor interaction, some cross-interactions between ligands and co-receptors able to activate RET have been described.

The function of the normal RET receptor has been intensely studied in neurons and in kidney development. In relation to human pathology, sporadic RET translocations have been implicated in the etiology of papillary thyroid carcinoma (RET/PTC). At least ten RET/PTC variants have been described in which the RET tyrosine-kinase domain is fused to other genes, although only RET/PTC1 (fused to the 5' end of the CCDC6 gene) and RET/PTC3 (fused with the NCOA4 gene) are common. More than 110 congenital RET mutations are also well known for causing two diseases in humans: Hirschprung's disease and Multiple Endocrine Neoplasia type 2 (MEN2). In Hirschprung's disease, or colonic aganglionosis, mutations are considered as inactivating RET function while in the MEN2 variants MEN2A, MEN2B and FMTC mutations are considered as activating RET function. However, it is not well explained how certain mutations can cause both Hirschprung's disease and MEN2.

In the adenopituitary, RET and the co-receptor GFRA1 is expressed exclusively in the somatotrophs secreting hGH (Garcia-Lavandeira M, et al. "Functional role of the RET dependence receptor, GFRA co-receptors and ligands in the pituitary"; Front. Horm. Res. 2010; 38: 127-138). Normal human and rodent somatotrophs express RET, GDNF and GFRA1, with RET working as a dependent receptor, a mechanism shared by a few other receptors. Thus, in the absence of GDNF its ligand RET is processed at the membrane by Caspase-3 and induces overexpression of PIT1, leading to induction of the CDKN2A/ARF promoter and ARF expression; p14ARF protein then binds to and inhibits MDM2, leading to p53 accumulation and apoptotic cell death. When GDNF is present, RET dimerizes, activating its tyrosine kinase which induces AKT and cell survival. Thus, somatotrophs rely on the presence of the RET ligand GDNF for survival. Immunohistochemical studies have shown that ACRO are the only pituitary adenomas expressing all three proteins, RET, GDNF and GFRA1 (Japon MA et al.: "Glial-derived neurotropic factor and RET gene expression in normal human anterior pituitary cell types and in pituitary tumors"; J Clin Endocrinol Metab 2002; 87:1879-1884). More recently, in vitro experiments in nine acutely dispersed ACRO have suggested that ACRO maintain the RET/PIT1/ARF/p53 apoptotic pathway and need GDNF expression to survive (Diaz-Rodriguez E et al.: "Somatotropinomas, but not Nonfunctioning Pituitary Adenomas, maintain a Functional Apoptotic RET/Pit1/ARF/p53 Pathway that is Blocked by excess GDNF"; Endocrinology 2014; 155:4329-4340). In a small group of ACRO tissues, GDNF expression was inversely correlated to PIT1 expression. To date, no studies have shown whether other ligands or GFRA receptors are expressed in ACRO, nor are there any data relating to the clinical relevance of the RET/PIT1/ARF/p53 apoptotic or the RET/GDNF survival pathways in acromegaly.

### SUMMARY OF THE INVENTION

The inventors investigated the role of the RET pathways in acromegaly, specifically in relation to clinical tumor characteristics, response to treatment and prognosis. They also carried out *in vitro* tests on five multikinase inhibitors able to act on the GDNF-induced RET dimerization/survival pathway. It was found that ARF expression, preferably measured at the time of surgery, is a good prognosis factor in acromegaly. Additionally, it was found that the RET/GDNF pathway plays an important role in acromegaly, which opens the door to new therapeutic approaches. The inventors found that Sorafenib, a RET/GDNF pathway inhibitor, is a potential therapeutic agent in resistant ACRO.

Therefore, in one aspect the invention is directed to an in vitro method for determining the prognosis of a patient suffering from acromegaly, comprising the steps of:
a. determining the expression level of ARF gene in a sample from said patient, and
b. comparing said expression level with a reference value,
wherein a decreased expression level of ARF gene compared to the reference value is indicative of bad prognosis, and
wherein an increased expression level of ARF compared to the reference value is indicative of a good prognosis.

In this method, preferably, a decreased expression level of ARF gene compared to the reference value is indicative of the presence of residual tumor after surgery and an increased expression level of ARF compared to the reference value is indicative of absence of residual tumor after surgery.

Additionally or alternatively, in this method a decreased expression level of ARF gene compared to the reference value is indicative of resistance to a therapy with an analogue of somatostatin (SSA) and an increased expression level of ARF compared to the reference value is indicative of a good response to a therapy with an analogue of somatostatin (SSA).The analogue of somatostatin is preferably a first-generation analogue of somatostatin (fgSSA), more preferably octeotride or lanreotride. Preferably the therapy with SSA is a therapy following surgery.

In the method of the invention the sample is a usually a pituitary sample, preferably a surgical sample.

In the method of the invention the determination of the expression level of the ARF gene is preferably carried out by determining the levels of mRNA.

In another aspect the invention is directed to an in vitro method for predicting the clinical response of a patient suffering from acromegaly to a treatment with a SSA comprising the steps of:
(a) determining the expression level of ARF gene in a sample from said patient and
(b) comparing said expression level with a reference value,
wherein a decreased expression level of ARF gene compared to the reference value is indicative of resistance to a therapy with an analogue of somatostatin (SSA) and wherein an increased expression level of ARF compared to the reference value is indicative of a good response to a therapy with an analogue of somatostatin (SSA).

In a further aspect, the invention is directed to an in vitro method for selecting a patient suffering from acromegaly for a therapy with a SSA, comprising the steps of:
(a) determining the expression level of ARF gene in a sample from said patient and
(b) comparing said expression level with a reference value,
wherein the patient is selected for said therapy if the expression level of ARF gene is increased compared to the reference value.

In another aspect, the invention is directed to an in vitro method for selecting a therapy for a patient suffering from acromegaly, comprising the steps of
(a) determining the expression level of ARF gene in a sample from said patient and
(b) comparing said expression level with a reference value,
wherein if the expression level of ARF gene is increased compared to the reference value, a therapy with a SSA is selected and
wherein if the expression level of ARF gene is decreased compared to the reference value, an alternative therapy is selected.

Preferably the alternative therapy is sorafenib.

The invention is also directed to a Tyrosine Kinase Inhibitor (TKI) that blocks the RET/GDNF survival pathway for use in the treatment of acromegaly.

It is preferably used when the acromegaly is resistant to first-line therapy following surgery, such as therapy with an analogue of somatostatin (SSA), preferably a fgSSA selected from octeotride or lanreotride.

Preferably the TKI is Sorafenib. Sorafenib is preferably administered in a dose below 800 mg/day, preferably below 600 mg per day, more preferably below 400 mg per day, and even more preferably below 200 mg/day.

In another aspect, the invention is directed to the use of a reagent capable of determining the expression level of ARF gene in a sample from a subject suffering from acromegaly for determining the prognosis of said patient, or for predicting the clinical response of a patient suffering from acromegaly to a treatment with a SSA, or for selecting a patient suffering from acromegaly for a therapy with a SSA.

### BRIEF DESCRIPTION OF THE FIGURES

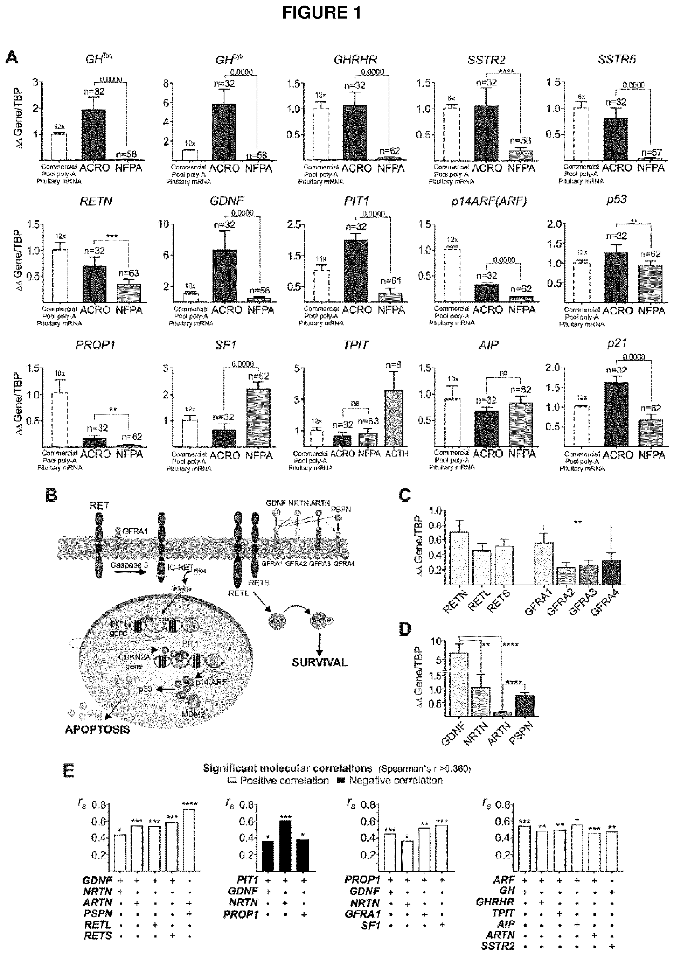

**Figure 1****: mRNA expression characterizing ACRO and the RET pathway.**
   **A)** Quantitative RNA expression comparing ACRO (n=32, blue bars) and NFPA (n=56-63, pink bars). In every sample, gene expression was normalized to a commercial pool of pituitary poly-A mRNA (technical control; white bars). Genes most highly expressed in ACRO were characteristic of functional pituitary somatotrophs, related to hGH secretion (GH^{Taq} (22 KDa), GH^{Sybr} (22, 20 and 17 KDa)), or PIT1 and hypothalamic regulation (GHRHR, SSTR2, SSTR5). However, the RET receptor (RETN), its ligand GDNF and genes in the RET pathway regulated at the RNA level (ARF and PIT1) were significantly more abundant in ACRO than NFPA. p53 was slightly upregulated in ACRO. SF-1 was characteristic of NFPA, and T-PIT of ACTH-secreting adenomas (orange bar).
   **B)** Cartoon representing isoforms of the RET receptor, differing at the C-terminal tail (long RETL and short RETS), its four ligands and its GFR-alpha co-receptors (GFRA1-4). Although there is preference of a ligand for a co-receptor, cross-interaction exists. In somatotrophs in the absence of ligand, RET is processed by Caspase-3 generating an intracellular fragment (IC-RET) that triggers a cell-death pathway through overexpression of PIT1 gene inducing p14ARF expression, p53 accumulation and apoptosis. When the ligand is present, RET dimerizes and activates its cytoplasmic tyrosine kinase activity leading to AKT phosphorylation and survival.
   **C)** In ACRO, both RETL and RETS are expressed in approximately equal amounts. Although all four RET co-receptors were expressed, the most highly expressed was GFRA1 (high affinity for GDNF), followed by GFRA4.
   **D)** GDNF was by far the most highly expressed ligand, with NTRN and PSPN also abundantly expressed.
   **E)** Significant correlations among all the genes studied in ACRO (rₛ>0·38) revealed that GDNF expression was significantly and positively correlated with expression of RET isoforms and the other ligands. GDNF (and NRTN) were negatively correlated with PIT1 expression but positively correlated with PROP1. As expected, expression of both transcription factors PIT1 and PROP1 were negatively correlated. PROP1 was also correlated with GFRA1 and SF1. ARF expression was significantly correlated with the somatotroph phenotype (GH, GHRHR, AIP), T-PIT and the ligand ARTN. (*, p<0-05; **, p<0·01; ***, p<0·001; ****, p<0·0001; 0·0000 means lower p).

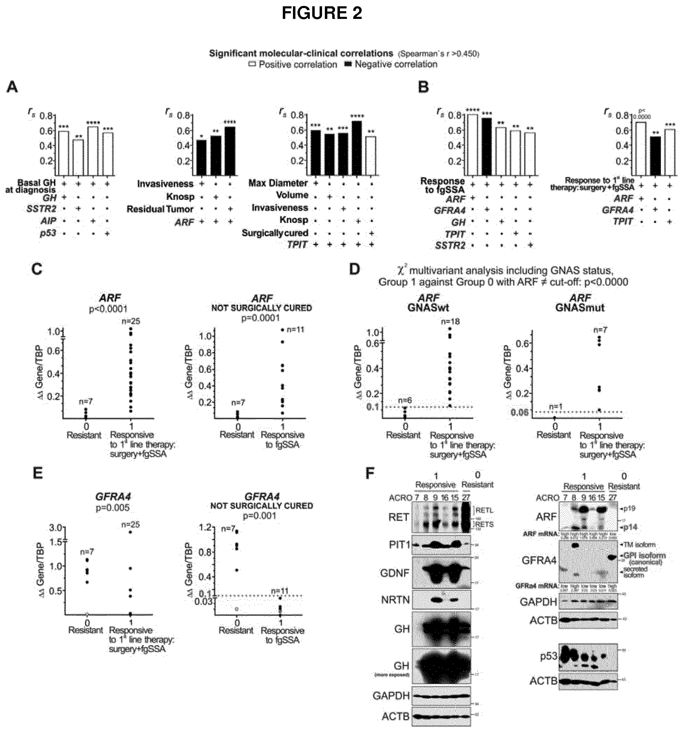

**Figure 2****: ARF mRNA expression as a prognostic marker for ACRO resistant** to **first-line combined treatment (surgery+fgSSA). GFRA4 as a prognostic** marker **only for those not cured by surgery.**
   **A-B:** Significant correlations (rs>0·5) between mRNA expression and clinical variables (diagnostic, pathologic and prognostic/follow-up).
   **A) Left:** serum hGH at diagnosis was correlated with hGH, SSTR2, AIP and p53. **Centre:** ARF was negatively correlated with the negative characteristics of the tumor (invasiveness, Knosp grade, residual tumor). **Right:** T-PIT was negatively correlated with negative tumor characteristics (diameter, volume, invasiveness and Knosp) but positively correlated with surgical cure.
   **B) Left:** ARF was the strongest positive marker for response to adjuvant treatment with fgSSA (rs>0·8). GFRA4 was the second-strongest marker but negatively correlated with response (rs>0·7). GH, TPIT and SSTR2 showed some positive correlation with response to analogs (rs 0·5-0·6). **Right:** Combining first-line treatments (surgery+fgSSA) into a single category, ARF (rs>0·7) and GFRA4 (rs=0·5) were the most significant positively and negatively correlated markers, respectively, followed by TPIT (rs=0·6) as a positive marker.
   **C-F:** Patients were categorized into two groups, Group 0 (Resistant: Not cured by surgery and resistant to analogs) and Group 1 (Responsive to first-line treatment, either cured by surgery or controlled with analogs). A subanalysis of patients not cured by surgery was also performed.
   **C)** ARF expression was a good discriminator of Group 0 and Group 1, both in the series as a whole (p<0·0001) and in patients not cured by surgery (p=0·0002).
   **D)** Samples were categorized with different cut-offs for non-mutated (GNASwt cut-off 0·1) or mutated (GNASmut, cut-off 0·06) GNAS; Chi-squared tests classified all samples.
   **E) Left:** Enhanced GFRA4 expression was not a good classifier for ACRO as some in Group 1 (Responsive, high ARF) had high GFRA4 expression. **Right:** In the group of non-surgically cured, GFRA4 was a good classifier in the opposite way to ARF, with high levels in Group 0 (Resistant to first-line therapy). Coloured dots show reoperations after radiotherapy (Group 0, yellow; Group 1, orange).
   **F)** Western blots of RET pathway (RET, PIT1, ARF, p53) and ligand (GDNF, NRTN) proteins with controls (GH, GAPDH, ACTB) from ACRO tissue extracts (Group 1 (Responsive) and Group 0 (Resistant)). ARF and GFRa4 protein levels corroborated mRNA levels described above. ACRO27 (Group 0) expressed high RET but low PIT-1 protein levels, correlating with absence of p14ARF and p53. GFRA4 showed three different molecular weights corresponding to distinct isoforms. The RET co-receptor (GPI, canonical isoform) was highly expressed in ACRO27 (Group 0) while ACRO7 and ACRO8 (Group 1) expressed different non-RET co-receptor isoforms. (*, p<0-05; **, p<0·01; ***, p<0·001; ****, p<0·0001; 0·0000 means lower p).

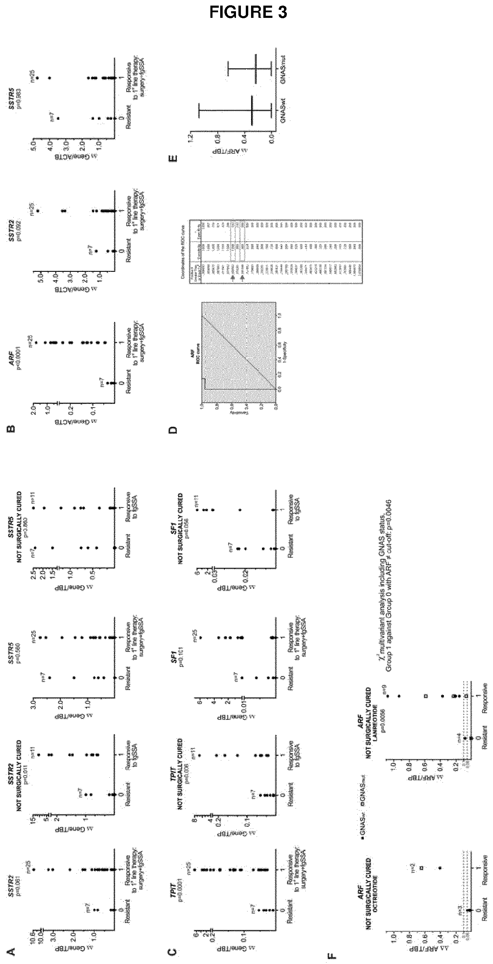

**Figure 3****: ARF mRNA expression cut-offs for identification of ACRO Responsive (Group 1) and Resistant (Group 0) to combined first-line treatment (surgery+fgSSA).** Patients were categorized into two groups, Group 0 (Resistant: not cured by surgery and resistant to analogs) and Group 1 (Responsive to first-line treatment, either cured by surgery or controlled with analogs). A subanalysis was also performed on patients not cured by surgery.
   **A)** Neither SSTR2 nor SSTR5 RNA expression was able to discriminate robustly between groups.
   **B)** Using b-actin (ACTB) as a control gene instead of TBP, ARF RNA expression also clearly discriminated between the two groups. Again, this did not occur with SSTR2 or SSTR5.
   **C)** T-PIT, a gene correlated with pathological tumor characteristics (see Figure 2A), could partly separate the groups of patients, although not as completely as ARF. SF1 could not separate groups convincingly.
   **D)** The Receiver Operating Curve (ROC) for ARF mRNA expression and separation of Group 0/ Group 1 shows a cut-off of 0·0595 (∼0·06), or a cut-off of 0·0915 (∼0·1) with 100% /85·7% specificity or 96%/100% sensitivity, respectively.
   **E)** Range and median of ARF expression in ACRO samples separated by GNAS mutation. ARF expression in GNASwt samples compared with GNASmut samples did not show differences in their medians but their range was wider, accounting for the slight difference in cut-off.
   **F)** Patients were separated by fgSSA treatment, Octeotride and Lanreotide, and ARF values plotted against response (Group 0, Group 1). The Lanreotide group was analysed by a multivariate analysis that included GNAS status (wtGNAS, cut-off>0·01; mutGNAS, cut-off>0·06).

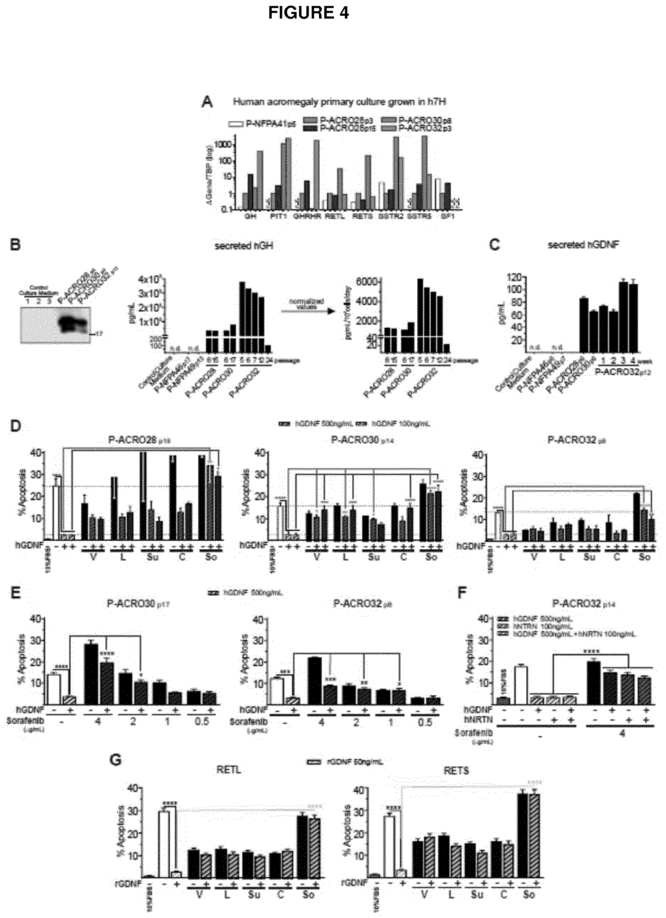

**Figure 4****: Primary cultures of human acromegaly in humanized conditions (h7H) unveiled Sorafenib as a potential new treatment.**
   **A)** Acromegaly (P-ACRO28, P-ACRO30 and P-ACRO32) and NFPA (P-NFPA41) cultured in h7H conditions grew for many passages while maintaining mRNA expression of key genes from Fig 1: in ACRO, GH, PIT1, GHRHR, RETL and RETS isoforms, SSTR2 and 5; in NFPA, SF1. Data are normalized to the first acromegaly P-ACRO28. Passage was performed by splitting the culture in two (shown by small 'p').
   **B)** Human GH (hGH) was secreted by cultured ACRO into the medium as demonstrated by western blot.
   **C)** Human GDNF (hGDNF) was secreted into the medium by ACRO, but not by NFPA, as demonstrated by ELISA. Secretion was increased as cells grew, as shown for ACRO32.
   **D)** When cells were deprived of GDNF, RET processing induced apoptosis (white bar) that was blocked by addition of GDNF (hatched white bars). Five TKIs used for other neuroendocrine tumors (Vandetanib (V), Lenvatinib (L), Sunitinib (Su), Cabozantinib (C) and Sorafenib (So)) were tested against the survival action of GDNF at clinically relevant concentrations. Three independent cultures are shown: P-ACRO28, P-ACRO30 and P-ACRO32. Some of the inhibitors enhanced RET-dependent apoptosis in the absence of GDNF (black bars) but did not have a strong effect on GDNF-induced survival (black hatched bars). Sorafenib was the only TKI that potently blocked the GDNF survival effect in the three ACRO without exacerbating RET apoptosis in the absence of GDNF (which we assumed to be a toxic effect).
   **E)** Dose-response curve of Sorafenib in P-ACRO30 and P-ACRO32, demonstrating a GDNF-counteracting effect at lower doses than those used in cancer treatment.
   **F)** Sorafenib could also block the survival effect of NRTN and combined GDNF+NRTN.
   **G)** Sorafenib could block the survival effect of GDNF on both human RETL and RETS isoforms, transfected in the non-RET-expressing rat pituitary somatotroph cell line GH4C1. (n.d.=not detected). (*, p<0·05; **, p<0·01; ***, p<0·001; ****, p<0·0001).

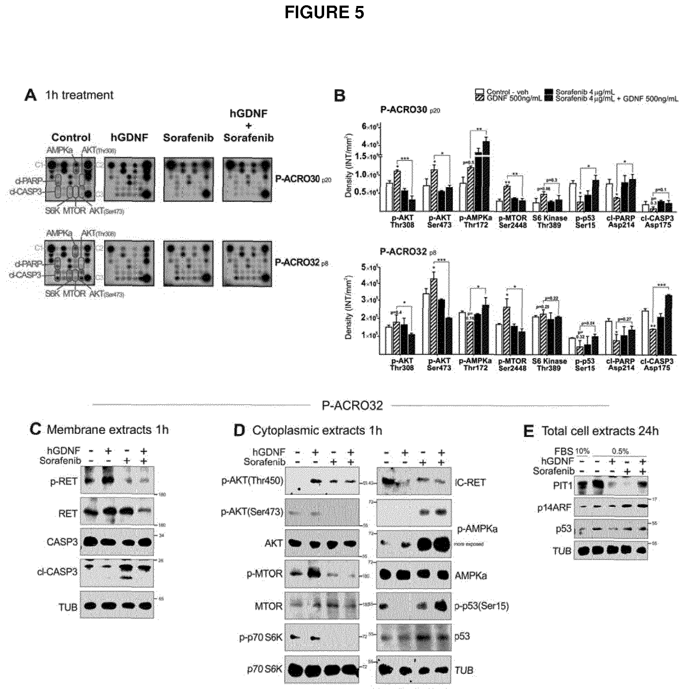

**Figure 5****: Blocking of RET signal transduction by Sorafenib exchanged the RET-GDNF survival pathway for the RET apoptotic pathway.**
   **A)** Antibody array performed with extracts from P-ACRO30 and P-ACRO32 following one- hour incubations in each of four conditions: deprivation (no GDNF), GDNF (500 ng/ml), Sorafenib (4 microg/ml) or Sorafenib+GDNF.
   **B)** Signal quantification: GDNF induced AKT/MTOR phosphorylation, and downregulated active p-p53 (Ser15 p-p53), as well as cleaved PARP (cl-PARP) and Caspase-3 (cl-CASP3), hallmarks of apoptosis. Sorafenib blocked the AKT pathway, inducing phosphorylation of AMPK, p53 and apoptotic markers.
   **C-E)** Western blots with different cell extracts confirmed the results of the array.
   **C)** At 1 hour in membrane extracts, GDNF induced full-length RET phosphorylation and blocked Caspase-3 cleavage; both were prevented by Sorafenib.
   **D)** At 1 hour in cytoplasmic extracts, GDNF phosphorylated AKT, mTOR and, less intensely, S6K while preventing p53 phosphorylation. In the presence of Sorafenib, GDNF was unable to activate the AKT/MTOR pathway, but induced phosphorylation of AMPK and p53. As expected, the caspase-processed cytoplasmic fragment of RET (IC-RET) was strongly expressed in deprived cells in the absence of GDNF, reduced in the presence of GDNF and recovered with Sorafenib.
   **E)** In cells deprived of GDNF for 24 hours, PIT1 was induced, activating ARF expression and p53 accumulation, thus leading to apoptosis. GDNF reduced PIT1, ARF and p53 expression, all of which recovered in the presence of Sorafenib. (*, p<0-05; **, p<0-01; ***, p<0·001).

### DETAILED DESCRIPTION OF THE INVENTION

By comparing the protein expression profile between a set of samples from acromegaly patients, the inventors of the present invention have determined the relevance of the RET receptor its ligands and co-factors in the survival and apoptotic pathways of somatotropes in acromegaly, and in resistance to established therapy. This allowed the identification of a survival pathway (RET/GDNF) and of a marker whose expression is a good indicator of the prognosis of the diseases, in particular following combined first-line therapy (surgery + treatment with SSA). Thus, the inventors have identified ARF as a biomarker the expression of which allows a solid prognosis of the disease (acromegaly), to select patients for a particular therapy, and also to select a therapy for a particular patient, depending on the levels of expression of ARF. Additionally, in view of the relevance of the RET/GDNF pathway in the survival of acromegaly somatotropic cells uncovered by the inventors, a new target and new therapies are provided for the treatment of acromegaly. The different aspects of the invention are described below.

### Method for determining the prognosis

In a first aspect, the invention relates to an *in vitro* method for determining the prognosis of a patient suffering from acromegaly, comprising the steps of:
a. determining the expression level of ARF gene in a sample from said patient, and
b. comparing said expression level with a reference value,
wherein a decreased expression level of ARF gene compared to the reference value is indicative of bad prognosis, and
wherein an increased expression level of ARF gene compared to the reference value is indicative of a good prognosis.

The term "method for determining the prognosis" or "prognostic method", as used herein, refers to the determination of the likelihood that an acromegaly patient will have a particular clinical outcome, whether positive (good prognosis) or negative (bad prognosis). In the present invention "clinical outcome" is understood as the expected course of a disease. It denotes the doctor's prediction of how a subject's disease will progress, and whether there is chance of recovery, disability or mortality.

In an embodiment, "good prognosis" refers to the prediction that the acromegaly patient will be responsive to combined first line therapy (combination of surgery and fgSSA).

In another embodiment, "good prognosis" refers to the prediction of a future absence of symptoms of acromegaly in a patient that has been treated by surgery. In another embodiment good prognosis refers to the absence of post-surgical residual tumor after surgery.

In a further embodiment, "good prognosis" refers to the prediction of a future good response to a therapy with SSA, in particular with first generation SSA such as octeotride or lanreotride.

In an embodiment, "bad prognosis" refers to the prediction that the acromegaly patient will not be cured by surgery and will be resistant to fgSSA during follow-up.

In another embodiment, "bad prognosis" refers to the prediction of a future resistance to a therapy with SSA, in particular with first generation SSA such as octeotride or lanreotride. In such a case, an alternative therapy can be administered, such as a TKI that blocks the RET/GDNF pathway, such as sorafenib, as explained below.

In a further embodiment "bad prognosis" refers to the presence of post-surgical residual tumor after surgery.

Residual tumor after surgery in the patient can be determined with usual imaging techniques used by the skilled person, such as MRI (molecular resonance imaging) or CT (computer tomography) scan.

The prognostic methods of the present invention can be used clinically to make treatment decisions by choosing the most appropriate treatment modalities for any particular patient. As will be understood by those skilled in the art, the prognosis of acromegaly, although preferred to be, need not be correct for 100% of the subjects to be diagnosed or evaluated. The term, however, requires that a statistically significant portion of subjects can be identified as having an increased probability of having a given outcome, as shown in the examples.

The person skilled in the art can easily determine if a part is statistically significant using several well-known statistical evaluation tools, for example, determination of confidence intervals, determination of p values, Student's t-test, Mann-Whitney test, etc. The details are found in Dowdy and Wearden, Statistics for Research, Wiley Interscience, New York, third edition (2004). The preferred confidence intervals are at least 50%, at least 60%, at least 70%, at least 80%, at least 90%, at least 95%, at least 97%, at least 98% or at least 99%. The p values are preferably 0.1, 0.05, 0.01, 0.005 or 0.0001. More preferably, at least 60%, at least 70%, at least 80% or at least 90% of the subjects of a population can be suitably identified by the method of the present invention. Any parameter which is widely accepted for determining prognosis of a patient can be used in the present invention including, without limitation, post-surgical residual tumor surgical cure or response to therapy with SSA during follow-up.

The term *"in vitro",* as used herein, refers to the fact that an experimental protocol or a method is not carried out on the body of a human subject, but on samples isolated from said subject, and already present in a laboratory tool, such as a test tube, dish or plate.

The term "acromegaly", as used herein, refers to a condition due to the production of too much growth hormone by the pituitary gland, often being released by a pituitary adenoma, after the end of adolescence.

The term "patient" or "subject", as used herein, refers to a human being of any race or age being diagnosed with acromegaly.

The term "treatment" or "therapy", as used herein, refers to any process, action, application, or the like, wherein a subject or patient, with a specific health condition or disorder is provided medical aid with the objective of improving the subject's condition, directly or indirectly, or slowing the progression of a condition or disorder in the subject, or ameliorating at least one symptom of the condition or disorder under treatment. In a preferred embodiment, a treatment or therapy refers to the administration at a specific regimen or dose for a given period of time, of a specific active agent, or a combination of active agents, comprised in a medicament or set of medicaments. In another embodiment, the therapy includes surgery to remove all or part of the pituitary adenoma.

The first step of the method of the invention comprises determining the expression level of the ARF gene in a sample of a patient to be tested.

The term ARF refers to an alternate reading frame protein product of the CDKN2A locus (i.e. INK4a/ARF locus). It is also called ARF tumor suppressor or p14ARF (in human). Its gene locus is on the short arm of chromosome 9p21 in humans. It is well known by the skilled person, further information on this protein and its encoding gene can be found in Ozenne P et al.: "The ARF tumor suppressor: structure, functions and status in cancer", Int. J. Cancer: 127, 2239-2247 (2010). The protein sequence for human ARF has the Uniprot accession number Q8N726 (13 February 2019).

The term "expression level", as used herein, refers to the expression level of a gene product, more specifically to a measurable quantity of a gene product produced by a specific gene in a specific sample of the subject. The term "gene product", as used herein, refers to a transcriptional product or a translational product, and thus corresponds to the mRNA transcribed from said gene or to the protein encoded by a specific gene. Thus, as understood by the person skilled in the art, the expression level of a gene product corresponds to a quantified amount of the protein encoded by said gene or to the quantified amount of the mRNA transcribed from said gene in a specific sample. When referring to the expression level of a biomarker, it is understood that the gene product is said biomarker, which, as indicated below, can be an mRNA or a protein transcribed from or encoded by the selected gene of the invention (in this case ARF).

The level of mRNA can be determined by methods well known in the art. For example the nucleic acid contained in the sample is first extracted according to standard methods, for example using lytic enzymes or chemical solutions or extracted by nucleic-acid-binding resins following the manufacturer's instructions. The extracted mRNA is then detected by hybridization (e.g., Northern blot analysis or by oligonucleotide microarrays after converting the mRNA into a labeled cDNA) and/or amplification (e.g., RT-PCR). Quantitative or semi-quantitative RT-PCR is preferred. Real-time quantitative or semiquantitative RT-PCR is particularly advantageous. Preferably, primer pairs are designed in order to overlap an intron, so as to distinguish cDNA amplification from putative genomic contamination. Suitable primers may be easily designed by the skilled person, as illustrated in the examples.

In order to normalize the values of mRNA expression among the different samples, it is possible to compare the expression levels of the mRNA of interest in the test samples with the expression of a control RNA. A "control RNA", as used herein, relates to RNA whose expression levels do not change or change only in limited amounts. Preferably, the control RNA is mRNA derived from housekeeping genes and which code for proteins which are constitutively expressed and carry out essential cellular functions. Preferred housekeeping genes for use in the present invention include 18-S ribosomal protein, β-2-microglobulin, ubiquitin, cyclophilin, GAPDH, PSMB4, tubulin and β-actin. In another preferred embodiment the control RNA is ACTB or TBP. TBP is preferably used as control of gene expression. It is considered as a gold standard gene as a control in quantitative mRNA assays since it is the gene presenting a more stable expression among different human tissues and cell types. The use of TBP as a control of gene expression is well known by the skilled person. The examples provide more information about this use of TBP.

The relative gene expression quantification may be calculated according to the comparative threshold cycle (Ct) method using a housekeeping gene as an endogenous control and commercial RNA controls as calibrators. Final results are determined according to the formula 2-(ΔCt sample-ΔCt calibrator), where ΔCt values of the calibrator and sample are determined by subtracting the Ct value of the target gene from the value of the control gene.

Other methods of amplification include ligase chain reaction (LCR), transcription-mediated amplification (TMA), strand displacement amplification (SDA) and nucleic acid sequence based amplification (NASBA). Preferably, the quantity of mRNA, or the mRNA level, is measured by quantitative or semi-quantitative RT-PCR or by real-time quantitative or semi-quantitative RT-PCR. Conventional methods for quantifying mRNA expression levels can be found, for example, in Sambrook et al., "Molecular cloning: A Laboratory Manual", fourth edition (2012) Cold 35 Spring Harbor Laboratory Press, N.Y., Vol. 1-3.

Alternatively, it is also possible to determine the expression level of a gene by means of the determination of the level of the protein encoded by said gene, since if the expression of the gene is increased, an increase of the amount of corresponding proteins occur and if the expression of the gene is decreased, a decrease of the amount of corresponding proteins occur. Therefore, the level of the ARF protein can be determined by any method known in the art suitable for the determination and quantification of a protein in a sample. By way of a non-limiting illustration, the level of a protein can be determined by means of a technique which comprises the use of antibodies with the capacity for binding specifically to the assayed ARF protein (or to fragments thereof containing the antigenic determinants) and subsequent quantification of the resulting antigen-antibody complexes, or alternatively by means of a technique which does not comprise the use of antibodies such as, for example, by techniques based on mass spectroscopy. The antibodies can be monoclonal, polyclonal or fragments thereof, Fv, Fab, Fab' and F(ab')2, scFv, diabodies, triabodies, tetrabodies and humanized antibodies. Similarly, the antibodies may be labelled. Illustrative, but non-exclusive, examples of markers that can be herein used include radioactive isotopes, enzymes, fluorophores, chemoluminescent reagents, enzyme cofactors or substrates, enzyme inhibitors, particles, or dyes. There is a wide variety of known tests that can be used according to the present invention, such as combined application of non-labelled antibodies (primary antibodies) and labelled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins, or determining the protein levels, include, for instance, affinity chromatography techniques or ligand-binding assays. Preferred methods to determine the quantity of a protein, or the protein level in a sample, are liquid chromatography followed by mass-spectrometry, or western blot.

The expression "reference value" of the expression level of a gene product or the "reference value" of the expression level of a biomarker, as used herein, refers to the expression level of a gene product or of a biomarker in one or more samples used as reference (and referred to as "reference samples") used to evaluate the expression level of a biomarker in a sample of interest (referred to as test sample). Said reference and test samples correspond to any of those defined herein when determining the expression level of a gene product or of a biomarker. As understood by a person skilled in the art, the reference and test samples should derive from the same type of body fluid or tissue.

The reference value or reference level can be an absolute value; a relative value; a value that has an upper or a lower limit; a range of values; an average value; a median value; a mean value; or a value as compared to a particular control or baseline value. A reference value can be based on an individual sample value or on a large number of samples, such as from population of subjects of the chronological age matched group, or based on a pool of samples.

Preferably the "reference sample" is a sample obtained from a subject not having acromegaly. As a person skilled in the art will understand, the reference expression level can also consist on the mean of expression levels determined in a group of reference samples as just defined. In fact, it is preferred to use the mean of a group of reference samples to reduce the variability due to the difference in sample donors (age, sex, etc.) and difference in the quality of the sample tissue obtained from the different donors.

In a preferred embodiment the reference sample is a human pituitary Gland pool obtained from a number of samples of individuals not suffering from acromegaly which is at least 5, preferably at least 10, more preferably at least 15, even more preferably at least 20. A number of samples above 30 or even above 50 is even more preferred to obtain the reference value.

It is also preferred that the pool of samples is enriched in mRNA transcripts. Commercial pools of human Pituitary Gland enriched in mRNA obtained from donors (usually after sudden death) are available from commercial providers and preferably used to obtain the reference value for the expression of ARF. The examples provide more information to the skilled person on how to obtain a reference value, and how to compare the expression levels of ARF with the reference value.

The term "sample", as used herein, refers to biological material isolated from a subject. The biological sample contains any biological material suitable for detecting RNA or protein levels of ARF. In a particular embodiment, the sample comprises genetic material, e.g., DNA, genomic DNA (gDNA), complementary DNA (cDNA), RNA, heterogeneous nuclear RNA (hnRNA), mRNA, etc., from the subject under study. The sample can be isolated from any suitable tissue or biological fluid such as, for example blood, saliva, plasma, serum, urine, cerebrospinal liquid (CSF), a surgical specimen, a specimen obtained from a biopsy, and a tissue sample embedded in paraffin.

In a preferred embodiment, the sample is a pituitary surgical sample obtained from a patient suffering from acromegaly. The sample is preferably a fresh sample, a frozen fresh sample or a sample embedded in paraffin. More preferably the sample is preferably a fresh sample obtained from surgery of a acromegaly patient. Preferably, the sample is pre-treated with RNAlater for better preservation of the RNA.

According to the prognostic method of the invention, the level or the expression level of ARF is considered "decreased" when the expression level of ARF in a sample is lower than its reference value. The expression level of ARF is considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100% lower than its reference value.

Likewise, in the context of the prognostic method of the invention, the expression level of RF is considered "increased" when the expression level of ARF in a sample is higher than its reference value. The expression level of ARF is considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

### Method for predicting the clinical response

In a second aspect, the invention relates to an *in vitro* method for predicting the clinical response of a patient suffering from acromegaly to a treatment with a SSA comprising the steps of:
(a) determining the expression level of ARF gene in a sample from said patient and
(b) comparing said expression level with a reference value,
wherein a decreased expression level of ARF gene compared to the reference value is indicative of resistance to a therapy with an analogue of somatostatin (SSA) and wherein an increased expression level of ARF compared to the reference value is indicative of a good response to a therapy with an analogue of somatostatin (SSA).

The term "predicting the clinical response", as used herein, refers to the likelihood that a patient will have a particular clinical outcome, whether positive or negative. The predictive methods of the present invention can be used clinically to make treatment decisions by choosing the most appropriate treatment modalities for any particular patient. The predictive methods of the present invention are valuable tools in predicting if a patient is likely to respond favorably to a treatment regimen. The prediction may also include prognostic factors. As it will be understood by those skilled in the art, the prediction, although preferred to be, need not be correct for 100% of the patients to be evaluated. The term, however, requires that a statistically significant portion of subjects can be identified as having an increased probability of having a given clinical response as described above.

The term bad response, as used herein, refers to not obtaining beneficial or desired clinical results which can include, but are not limited to, alleviation or amelioration of one or more symptoms or conditions, diminishment of extent of disease, stabilized (i.e. not worsening) state of disease, delay or slowing of disease progression, amelioration or palliation of the disease state, and remission (whether partial or total), whether detectable or undetectable.

All the particular terms and embodiments of the terms defined above in connection with the prognostic method of the invention also apply to the second method of the invention.

Native somatostatin, also known as growth hormone-inhibiting hormone (GHIH), has a very short serum half-life because of the enzymatic degradation. However, more potent and long-acting analogs of somatostatin have been developed and they are biologically stable. The term somatostatin analogue (SSA) refers to compounds having somatostatin pharmacological action. First generation somatostatin analogues (fgSSA) are compounds such as octeotride and lanreotide. Other SSA are pasireotide (Signifor®, Signifor LAR®) and vapreotide (Sanvar®).

In the methods of the invention the SSA is preferably octreotide or lanreotide.

Octreotide (Sandostatin®) is a long-acting somatostatin analogue. It is usually administered in a long-acting repeatable (LAR) formulation, formed by incorporating octreotide into microspheres of a biodegradable polymer and used as a monthly injection. There are three dosage forms used in clinical routine practice: 10 mg, 20 mg, and 30 mg.

Lanreotide (Somatuline®) binds to the same receptors as somatostatin, although with higher affinity to peripheral receptors, and has similar activity. Lanreotide has a much longer half-life than somatostatin, and produces far more prolonged effects. Lanreotide is available in two formulations: a sustained release formulation Somatuline LA), which is injected intramuscularly every ten or fourteen days, and an extended release formulation (Somatuline Autogel or Somatuline Depot) which is administered subcutaneously once a month.

### Method for selecting a patient for a therapy

In a third aspect, the invention relates to an *in vitro* method for selecting a patient suffering from acromegaly for a therapy with a SSA, comprising the steps of:
(a) determining the expression level of ARF gene in a sample from said patient and
(b) comparing said expression level with a reference value,
wherein the patient is selected for said therapy if the expression level of ARF gene is increased compared to the reference value.

The expression "selecting a patient for a therapy", as used herein, relates to the identification of a patient for a therapy designed to cure a disease or palliate the symptoms associated with the disease, in this case acromegaly. As explained above and understood by those skilled in the art, the selection of a patient, although preferred to be, need not be adequate for 100% of the subjects selected according to the third method of the invention. The term, however, requires that a statistically significant portion of subjects were correctly selected. Whether the selection of a patient in a population of subjects is statistically significant can be determined by the person skilled in the art using well known statistic evaluation tools, as described above. The preferred confidence intervals are at least 50%, at least 60%, at least 70%, at least 80%, at least 90%, at least 95%, at least 97%, at least 98% or at least 99%. The p values are preferably 0.1, 0.05, 0.01, 0.005 or 0.0001. More preferably, at least 60%, at least 70%, at least 80% or at least 90% of the subjects of a population can be suitably identified by the method of the present invention.

All the particular terms and embodiments of the terms defined above in connection with the prognostic method of the invention or the method for selecting a therapy of the invention apply to the third method of the invention.

### Method for selecting a therapy for a patient suffering from cancer

In a fourth aspect, the invention relates to an *in vitro* method for selecting a therapy for a patient suffering from acromegaly, comprising the steps of
(a) determining the expression level of ARF gene in a sample from said patient and
(b) comparing said expression level with a reference value,
wherein if the expression level of ARF gene is increased compared to the reference value, a therapy with a SSA is selected and
wherein if the expression level of ARF gene is decreased compared to the reference value, an alternative therapy is selected.

The term "selecting a therapy for a patient", as used herein, relates to the identification of a therapy or treatment to cure acromegaly or palliate the symptoms of acromegaly in a particular patient.

The term "alternative therapy", as used herein, means an effective therapy acromegaly which is not a treatement with a SSA.

In a particular embodiment, the alternative therapy is a Tyrosine Kinase Inhibitor (TKI) that blocks the RET/GDNF survival pathway, preferably sorafenib, as explained below.

All the terms and particular embodiments of these terms defined above in connection with the first, second and third methods of the invention apply to the fourth method of the invention.

### Therapeutic method

In a fifth aspect, the invention relates to a method for the treatment of acromegaly in a patient affected by this condition, comprising administering a therapeutically effective amount of a Tyrosine Kinase Inhibitor (TKI) that blocks the RET/GDNF survival pathway.

Alternatively, the invention relates to a Tyrosine Kinase Inhibitor (TKI) that blocks the RET/GDNF survival pathway for use in the treatment of acromegaly.

Alternatively, the invention relates to the use of a Tyrosine Kinase Inhibitor (TKI) that blocks the RET/GDNF survival pathway for the preparation of a medicament for the treatment of acromegaly.

The RET/GDNF survival pathway blocks the apoptotic pathway in which RET, in the absence of GDNF, induces Pit-1 overexpression, leading to increased p53 expression and apoptosis.

The RET/GDNF survival pathway is the pathway in which RET, in the presence of GDNF, dimerizes, activating its tyrosine kinase which induces AKT and cell survival. Specifically, a GDNF/GFRα1/Ret complex induces PI3K activation, leading to Akt phosphorylation and activation, thus preventing apoptosis.

By inhibiting this pathway with a TKI, survival of somatotrophs is avoided which would result in an effective therapy for acromegaly. Methods for determining if an agent blocks the RET/GDNF survival pathway are widely known.

Preferably, in the therapeutic method of the invention the TKI is sorafenib.

The term "Sorafenib" refers to the small molecular inhibitor 4-[4-[[4-chloro-3-(trifluoromethyl)phenyl]carbamoylamino]phenoxy]-N-methyl-pyridine-2-carboxamide. Sorafenib and the preparation thereof are described inter alia in WO 00/42012 and WO 03/068228. It is understood that pharmaceutically acceptable salts of Sorafenib as well as commonly used prodrugs are also within the scope of the invention.

Sorafenib is preferably administered at doses below the standard doses (800 mg/day). Preferably it is administered in a dose below 600 mg per day, more preferably below 400 mg per day, and even more preferably below 200 mg/day. This allows to obtain the desired therapeutic effect without the inconvenient or reduction of the secondary effects of sorafenib.

The effective amount of a Tyrosine Kinase Inhibitor (TKI), preferably Sorafenib, may be administered, for example, by systemic (e.g intravenous, subcutaneous, intramuscular injection), oral, parenteral or topical administration. Sorafenib is usually administered orally, which makes it convenient for the patient.

Preferably, sorafenib is administered as alternative therapy to a patient with a poor prognosis as determined by the methods of the invention, or to a patient resistant to resistance to a therapy with a SSA.

### Uses of the invention

In a sixth aspect, the invention relates to the use of a reagent capable of determining the expression level of ARF gene in a sample from a subject suffering from acromegaly for determining the prognosis of said patient, or for predicting the clinical response of a patient suffering from acromegaly to a treatment with a SSA, or for selecting a patient suffering from acromegaly for a therapy with a SSA.

The term "reagent capable of determining the expression level of ARF gene", as used herein, refers to any reagent suitable for detecting the gene product produced by ARF gene, wherein the gene product can be a transcriptional product (mRNA) or a translational product (ARF protein encoded by the ARF gene).

In a particular embodiment, the reagent capable of determining the expression level of ARF is a nucleic acid capable of hybridizing specifically with the ARF mRNA. Nucleic acids capable of hybridizing specifically with the ARF mRNA are, for example, one or more pairs of primer oligonucleotides for the specific amplification of fragments of the mRNA (or their correspondent cDNA) of ARF and/or one or more probes for the identification of ARF mRNA. As the skilled person understands, the oligonucleotide primers and probes can be used in all techniques of gene expression profiling (RT-PCR, SAGE, TaqMan, Real Time-PCR, FISH, NASBA, etc).

In a preferred embodiment the reagent capable of determining the expression level of ARF are primer oligonucleotides, such as those described in the examples of the invention.

In another particular embodiment, the reagent capable of determining the expression level of ARF is an antibody that specifically binds to the ARF protein encoded by ARF gene. Antibodies, or fragments thereof, capable of specifically binding to ARF protein or to variants thereof are, for example, monoclonal and polyclonal antibodies, antibody fragments, Fv, Fab, Fab' and F(ab')2, ScFv, diabodies, triabodies, tetrabodies and humanised antibodies. These antibodies can be used in conventional methods for detecting protein expression levels, such as Western-blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips, protein microarrays including specific antibodies or assays based on colloidal precipitation in formats such as dipsticks, etc.

All the terms and particular embodiments of these terms defined above in connection with the previous methods of the invention apply to the sixth method of the invention.

The invention is detailed below by means of the following examples, which are merely illustrative and by no means limiting for the scope of the invention.

### EXAMPLES

### A. MATERIALS AND METHODS

### Study design and Clinical data

The study followed the European Convention on Human Rights and Biomedicine (ETS n°164) reflected in the Spanish Law of Research in Biomedicine (14/2007). It is a prospective study including acromegaly (ACRO) patients from October 2010 till February 2017 from three hospitals in Spain. The study was approved nationally by the Ethics Committee of REMAH at Hospital Virgen del Rocio (Sevilla) (Approval number: 1/2010) and locally by the Ethics Committee in the Complejo Hospitalario Universitario de Santiago de Compostela (CHUS), where the molecular analyses were performed. Specific consent was obtained from every participant. An approved written consent was obtained from each patient participating in the study. The patients were operated by endoscopic endonasal transphenoidal surgery. Non-functioning pituitary adenomas (NFPA) were also prospectively collected in Santiago (N= 50) and Leon (N= 13) for comparison to ACRO at the molecular level. Tissue samples were obtained from the pituitary adenomas at the time of surgery. The study was designed following REMARK guidelines.

N≥5 was calculated as the sample size in a group to obtain significant differences. Sample size was calculated based on the known failure of first-line therapy: surgery failure is reported in series varying from 60% to 35%; of the remaining non-surgically cured patients, half will not respond to therapy with fgSSA; that is from 30% to 17·5% of the patients will be Resistant. That means that to have N≥5 in the Resistant group the study must have a total size of 17-29 patients. We included 30 to be in the safe side.

Molecular data were obtained from the pituitary tumor surpluses in a single laboratory of Santiago de Compostela.

### Tissue sample management

Adenoma tissue surplus was immersed directly in RNAlater (AM7021, Ambion, Netherlands), kept at 4° C for 24 hours -during which time they were couriered to Santiago de Compostela, and kept at -20° C until further use. In some samples, when the surplus tissue was >10 mg, a small piece was transferred to the culture room to perform h7H primary culture (see below).

### RNA and DNA extraction

Nucleic acids were extracted with AIIPrep DNA/RNA Mini Kit (80284, Qiagen, Germany) were DNA is first purified by affinity column, and RNA is purified from the eluted fraction in a second affinity column including treatment with RNasa-Free DNase Set (79254, Qiagen, Germany). DNA was QC and quantified Quantifiler Human DNA kit (4343895, Applied Biosystems, UK). RNA was QC and quantified by Nanodrop 2000 (Thermo).

### qRT-PCR assay

500 ng of total RNA were incubated with 0.5 IU RNAse free DNAse I (EN0521, Thermo), 1 microL 10X buffer and water till 10 microL at 37° C for 30 minutes. Reaction was inactivated by adding 1 microL 50 mM EDTA and heating at 65° C for 10 minutes. cDNA was obtained after adding to this tube 1.5 microL with 300 IU MMLV (28025-013, Invitrogen, USA) with 6 microL 5X First-Strand Buffer and, following supplier protocol, including 1·5 microL de dNTPs 10mM, 0·1 microL Random Primers, 3 microL 0·1M DTT, 1ul RNaseOUT™ Recombinant Ribonuclease Inhibitor (40 units/µL) and H2O in a 30 microL reaction.

50ng, 25ng and 12·5ng of a commercial human Pituitary Gland Poly A+ mRNA pool (1305204A, Clontech, USA) was similarly reverse-transcribed in three independent reactions to use as a technical control. Expression was detected by qPCR using 1 microL of the cDNA reaction plus 6 microL 2x TaqMan Gene Expression MasterMix (4369016 Applied Biosystems) and 6 microL diluted primers in 96 wells plates in a 7500 Real-Time PCR System (4351105, Applied Biosystems, USA). For primers, when possible we used commercial Taqman Gene expression primer sets (Applied Biosystems) as listed in Table 1:

| **Table 1: qRT-PCR and Sanger Primers** | | | |
|---|---|---|---|
| **Gene** | **Sequence (5'→3')** | **Amplicon** | **Annealing T^{a}** |
| **TBP** | Fw: 5'-GCCCGAAACGCCGAATAT-3 (SEQ ID NO:1) | 67 bp | 60°C |
| | Rw: 5'-TTCGTGGCTCTCTTATCCTCATG-3' (SEQ ID NO:2) | | |
| | Pb: 5'FAM-TCCCAAGCGGTrTGCTGCGGTA-3'TAM (SEQ ID NO:3) | | |
| **ACTB** | *Applied Biosystems:* Hs99999903_ml | 171 bp | 60°C |
| **GH1**^{Taq} | *Applied Biosystems:* Hs00236859_ml | 82 bp | 60°C |
| **GH1**^{Syb} | Fw: *5*'*-GACCTAGAGGAAGGCATCCAAA*-3' (SEQ ID NO:4) | 143 bp | 60°C |
| | Rw: 5'-AGCAGCCCGTAGTTCTTGAGTAG-3' (SEQ ID NO:5) | | |
| **PRL** | *Applied Biosystems:* Hs00168730_ml | 76 bp | 60°C |
| **POMC** | *Applied Biosystems:* Hs01596743_ml | 142 bp | 60°C |
| **LHB** | Fw: 5'*-GCCTCCTCTTCCTCTAAAGACC-*3'(SEQ ID NO: 6) | 104bp | 60°C |
| | Rw: 5'*-GCGGATTGAGAAGCCTTTATT-*3'(SEQ ID NO: 7) | | |
| **FSHB** | Fw: 5'-TTGGTGTGCTGGCTACTGCT-3'(SEQ ID NO: 8) | 115bp | 60°C |
| | Rw: 5'-GGGCACTCTCACTGTTTCGT-3'(SEQ ID NO: 9) | | |
| **LHB** | Fw: 5'-*GCCTCCTCTTCCTCTAAAGACC*-3'(SEQ ID NO: 10) | 104 bp | 60°C |
| | Rw: 5'-*GCGGATTGAGAAGCCTTTATT-*3'(SEQ ID NO: 11) | | |
| **FSHB** | Fw: 5'*-TTGGTGTGCTGGCTACTGCT*-3' (SEQ ID NO: 12) | 115 bp | 60°C |
| | Rw: 5'-*GGGCACTCTCACTGTTTCGT-*3' (SEQ ID NO: 13) | | |
| **CGA** | *Applied Biosystems: Hs00174938_m1* | 85 bp | 60°C |
| **GHRHR** | *Applied Biosystems:* Hs00173457_m1 | 104 bp | 60°C |
| **SSTR2** | Fw: 5'-*GGCATGTTTGACTTTGTGGTG-*3' (SEQ ID NO: 14) | 185 bp | 60°C |
| | Rw: 5'*-GTCTCATTCAGCCGGGATTT*-3'(SEQ ID NO: 15) | | |
| **SSTR5** | Fw: 5'*-CTGGTGTTTGCGGGATGTT*-3'(SEQ ID NO: 16) | 183 bp | 60°C |
| | Rw: 5'- GAAGCTCTGGCGGAAGTTGT-3' (SEQ ID NO: 17) | | |
| **RETN** | *Applied Biosystems:* Hs01120030_m1 | 83 bp | 60°C |
| **RETL** | *Applied Biosystems: Hs04259657_s1* | 110 bp | 60°C |
| **RETS** | *Applied Biosystems: Hs04259656_s1* | 96 bp | 60°C |
| **GFRA1** | *Applied Biosystems: Hs00237133_m1* | 69 bp | 60°C |
| **GFRA2** | *Applied Biosystems: Hs00176393_m1* | 88 bp | 60°C |
| **GFRA3** | *Applied Biosystems: Hs00181751_m1* | 89 bp | 60°C |
| **GFRA4** | *Applied Biosystems: Hs00360831*_*g1* | 152 bp | 60°C |
| **GDNF** | *Applied Biosystems:* Hs01931883_s1 | 91 bp | 60°C |
| **ARTN** | *Applied Biosystems: Hs00754699_s1* | 78 bp | 60°C |
| **NRTN** | *Applied Biosystems: Hs00177922_m1* | 80 bp | 60°C |
| **PSPN** | *Applied Biosystems: Hs00358822_g1* | 92 bp | 60°C |
| **P14ARF** | *Applied Biosystems: Hs00924091_m1* | 84 bp | 60°C |
| **p53 (TP53)** | *Applied Biosystems: Hs01034249*_*m1* | 108 bp | 60°C |
| **PIT1** | *Applied Biosystems: Hs00230821_m1* | 81 bp | 60°C |
| **TPIT** | *Applied Biosystems: Hs00193027_m1* | 65 bp | 60°C |
| **PROP1** | *Applied Biosystems: Hs00395073_m1* | 88 bp | 60°C |
| **SF1** | *Applied Biosystems: Hs00610436_m1* | 71 bp | 60°C |
| **AIP** | *Applied Biosystems: Hs00610222_m1* | 56 bp | 60°C |
| **p27 (CDKN1B)** | Fw: 5'*-GCAACCGACGATTCTTCTACTCAA-3'*(SEQ ID NO: 18) | 119 bp | 60°C |
| | Rw: *5'-CTTCTGAGGCCAGGCTTCTTG-3'(SEQ* ID NO: 19) | | |
| | Pb: 5'FAM-*TTTCAGACGGTTCCCCAAATGCCG-*3'TAM (SEQ ID NO: 20) | | |
| **p21 (CDKN1A)** | *Applied Biosystems: Hs00355782_m1* | 66 bp | 60°C |
| **GNAS** | Fw: *5'-GGACTCTGAGCCCTCTTTCC-3'(SEQ* ID NO: 21) | 351 bp | 55°C |
| | Rw: 5'*-CACAGCATCCTACCGTTGAA-3'*(SEQ ID NO: 22) | | |

When isoforms or high homology genes was an issue, that is for FSHB, LHB, SSTR2 and SSTR5, we used previously validated primers (see table 1) (Luque RM et al.: "The Molecular Registry of Pituitary Adenomas (REMAH): A bet of Spanish Endocrinology for the future of individualized medicine and translational research", Endocrinol Nutr 2016; 63:274-284) and instead 6 microL Brilliant III Ultra-Fast SYBR MasterMix (600882, Agilent Technologies, USA). hGH isoforms mRNA expression was detected using both assays. Each 96 well plate was used for a single gene reaction including duplicate samples of a group of 10 ACRO, 20 NFPA together with technical positive controls, negative control (ACRO9 reverse-transcribed in the absence of MMLV) and blank (all reagents without cDNA).

As control of general gene expression per sample, we used TBP based in published works (Radonic A, Tet al.: "Guideline to reference gene selection for quantitative real-time PCR", Biochem Biophys Res Commun 2004; 313:856-862; Lossos ISet al.: "Optimization of quantitative real-time RT-PCR parameters for the study of lymphoid malignancies"; Leukemia 2003; 17:789-795; Garcia-Rendueles A et al.: "Rewiring of the apoptotic TGF-beta-SMAD/NFkappaB pathway through an oncogenic function of p27 in human papillary thyroid cancer", Oncogene 2017; 36:652-666) and our previous experience (Garcia-Rendueles A et al.: "Rewiring of the apoptotic TGF-beta-SMAD/NFkappaB pathway through an oncogenic function of p27 in human papillary thyroid cancer"; Oncogene 2017; 36:652-666).

ACTB is not considered a good control because presents higher variability among samples from the same and different tissues. TBP is considered as a gold standard gene as a control in quantitative mRNA assays since it is the gene presenting a more stable expression among different human tissues and cell types and was used. ΔCt method was used to express results of each gene (ΔCt_{gene} =Ct_{gene} - Ct_{TBP}).

We calculated some data in parallel using TBP and ACTB obtaining similar results and statistical significance.

Undetectable samples in a specific gene (Ct_{gene}>40) were classified according to the CT_{TBP}. If any other sample with a similar Ct_{TBP} had a detectable Ct_{gene} (Ct gene <40), the sample was considered a very low expressing for that particular gene and its Ct gene transformed to 42 (two cycles over the limit). If the sample had a ΔCt_{TBP} over any other sample, the sample was considered a real technical undetectable sample for that gene (loss sample). The media of the three concentrations of the technical control (commercial human pituitary Gland pool) was considered as 1. The ΔΔCt method, ΔCt_{gene} -ΔCt_{gene-technical} control, was used to calculate the end value of the qPCR in each sample. Potency of 2^{-ΔΔCt} was the end result used for statistical comparisons.

### GNAS mutational profile and STRs analysis

GNAS gene was amplified from 50 ng DNA by PCR using 0·5 IU DreamTaq DNA Polymerase (EP0701 Thermo Scientific), 2·5 microL DreamTaq Buffer, 0·5 microL 10mM dNTPs, 0·5 microL 5microM Forward Primer and 0·5 microL 5 microM Reverse Primer and dH20 up to 25 microL. PCR Product was Sanger sequenced to check for mutations in nucleotide C601/codon R201 or nucleotide A680/codon Q227.

For assessment of similarity between pituitary tissue and primary culture DNA we used a combined panel of 16 STRs AmpFiSTR NGM Select kit (4457889, Applied Biosystems, Germany) including Identifiler Plus Panel V1 (Applied Biosystems) as pattern for the reading out. The obtained 16 STRs profiles were compared to the 8 STRs American Type Culture Collection (ATCC) database using ATCC Match algorithm according to ICLAC standards. (https://www.lgcstandards-atcc.org/en/STR_Database.aspx).

### h7H cell culture

Culture was performed as described (Bravo SB et al: "Humanized medium (h7H) allows long-term primary follicular thyroid cultures from human normal thyroid, benign neoplasm, and cancer"; J Clin Endocrinol Metab 2013; 98:2431-2441) except that the hormone mix was composed of insulin, cortisol, T3, GHRH, ghrelin, somatostatin, EGF, bFGF and glucagon.

The excised fragment of the adenoma surplus was digested in 1mg/ml collagenase IV (C9891-AG, Sigma, Israel) and 1X trypsin (T4674, Sigma, USA) in PBS for 45 min, and then filtered into complete h7H medium (including serum) through a 41-micron filter (NY4100010, Merck Millipore, Ireland) to remove undigested tissue fragments. Cells were washed several times with complete h7H medium and then seeded on one well of a 12-well TC-treated polystyrene plasticware (Costar, Thermo Scientific, USA). Every two weeks, cells were trypsinized and diluted 1:2. We performed the h7H culture in 11 NFPA and 4 ACRO, on whom 8 NFPA y 3 ACRO had grown. We were able to keep them for at least 25 passages.

Rat GH4C1 somatothroph cell line was cultured in DMEM (D6046, Sigma, UK) containing 10% FBS (Diaz-Rodriguez E et al.: "Somatotropinomas, but not Nonfunctioning Pituitary Adenomas, maintain a Functional Apoptotic RET/Pit1/ARF/p53 Pathway that is Blocked by excess GDNF"; Endocrinology 2014; 155:4329-4340). Since no STR profile has been standardized for rat lines, expression profile was used to assess phenotype. Transfection of RETL or RETS was performed using Nucleofector II (AAD-1001S, Amaxa, Germany) with the program A-20 and the L-kit (VCA-1005, Lonza, Germany) as described (Diaz-Rodriguez E et al. "Direct promoter induction of p19Arf by Pit-1 explains the dependence receptor RET/Pit-1/p53-induced apoptosis in the pituitary somatotroph cells"; Oncogene 2012; 31:2824-2835).

### Evaluation of anti-survival action of tyrosine kinase inhibitors (TKIs) activity

TKIs (TINIB tools, Czeck Rep) stocks were prepared in DMSO and kept at -20 °C. An essential step in our assay was deciding which TKIs concentrations tested *in vitro* were really relevant for their *in vivo* action. TKIs concentrations were based on the United States Food and Drug Administration (FDA) approved pharmacological review of each drug collecting data from Phase II-III clinical trials. We selected the mean concentration at steady-state (Cm_{SS}) detected in treated patients' serum.

h7H-primary pituitary acromegaly cells were seeded (10.000/well in mw48 for apoptosis; 50.000/mw12 for RNA or protein extracts) in full h7H medium including 10% serum. Two days later, cells were washed and medium changed to deprived medium (0·5% FBS and no hormones or additives). GDNF (100 or 500 ng/ml, stock: 10 microg/ml in PBS containing 0·1%BSA; 345872, Calbiochem, Israel), NRTN (100 ng/mL; stock: 10 microg/ml in PBS containing 0·1%BSA; 1297-NE-025, R&D Systems, USA) or vehicle (PBS containing 0·1%BSA) was added for 24 hours. A full h7H medium (with serum and additives) condition was included as a control of basal apoptosis. Appropriate TKIs dilutions or DMSO was added for the corresponding time with/without GDNF/NRTN.

For GH4C1 pituitary cells, 25000 transfected cells/well were seeded in a 48-well dish in full medium. The following day, cells were washed and cultured in DMEM+0·1% BSA for 48 hours with/without rat GDNF (50 ng/ml PF039-10ug, Calbiochem, USA) and TKIs.

### Apoptosis assay

Medium containing GDNF, TKIs, or vehicles was added for 24 hours followed by addition of 5 microM Hoescht-33258 (B1155, Sigma, USA) for 1 hour. Apoptosis was measured by live microscopy in an IX51 equipped with CellSens software (Olympus). At least six fields including 75-100 cells were registered per well detecting condensed brilliant blue (apoptotic cells) and homogeneous soft blue (live cells).

### 16-pad slide array

h7H-primary pituitary acromegaly cells were seeded and treated as described above for one hour. Cells were washed with ice-cold PBS and lysed with 30 microL of ice-cold Cell Lysis buffer (#7018, Cell Signaling Technology, USA). MicroBCA Protein Assay (23235 Thermo IL USA) was used to measure protein concentration. The 16-pad PathScan Intracellular Signaling array kit (#7323, Cell Signaling Technology, USA) was performed according to the manufacturer's instructions. In brief, the array was blocked using the array blocking buffer for 15 min at room temperature. 60 microg cell lysates/100 microL Array Diluent Buffer was then added and incubated for 2 hours at room temperature on an orbital shaker. After washing, Detection Antibody cocktail was added and incubated for 1 hour at room temperature. After washing, HRP-linked streptavidin was added and incubated for 30 min at room temperature. After washing, the Array glass slide was finally covered with 1X LumiGLO©/Peroxide solution and exposed to films in a linear time-course. The average pixel density of each marker was scanned and quantified by Quantity One (Bio-Rad) and normalized to pixel densities of the positive control spots.

### Cell extracts and Western blot

A small fragment of frozen acromegaly tissue was mixed with 100 microL lysis buffer (0·05 M Tris-HCl, 0·01 M EGTA, 1 mM EDTA, 16 mM Triton X-100, 1 mM sodium orthovanadate, 0·05 M sodium fluoride, 0·01 M sodium pyrophosphate and 0·25 M sucrose, adjusted to 7·5 pH; all of them from Sigma, USA, including freshly added Complete EDTA-free protease inhibitor cocktail tablets 11836170001, Roche Diagnostics, USA). A Pit-Mill tissue lyser (85300 Qiagen, Germany) was used for tree minutes at 20Hz. Lysates were centrifuged at 12000 rpm for 30 min and supernatants stored at -80°C.

Cell lysates obtained above for the 16-path slide array were considered cytoplasmic. For harder extraction including membrane proteins 30 microL of ice-cold Triton lysis buffer (50 mM Hepes pH 7·5, 1% Triton X-100, 150 mM NaCl, 5 mM EGTA, 1·5 mM MgCL, 10 mM Na pyrofosfate, 92 microg/mL Na₃VO₄, 2 microg/ml aprotinin and 4 mM PMSF, all from SIGMA) incubated for 20 min on ice. For total cell extracts 15 microL of boiling 1% SDS/well were added, scraped and boiled at 95 °C for 5 min in a thermoblocker, and finally diluted 1:3 with Triton lysis buffer. All lysates were centrifuged at 12000 rpm for 10 min and supernatants stored at -80°C.

MicroBCA Protein Assay (23235 Thermo IL USA) was used to measure protein concentration. 15-20 microg of protein extracts in 10-15% SDS-PAGE and electrotransferred to a PVDF membrane (Immun-Blot PVDF 162-0177; Bio-Rad, USA) with a semidry blotter. Membranes were blocked with 0·2% Tropix i-block (T2015, Applied Biosystems, USA) in 1X TBS for 2 hours. Antibodies and dilutions are showed in the following Table 2:

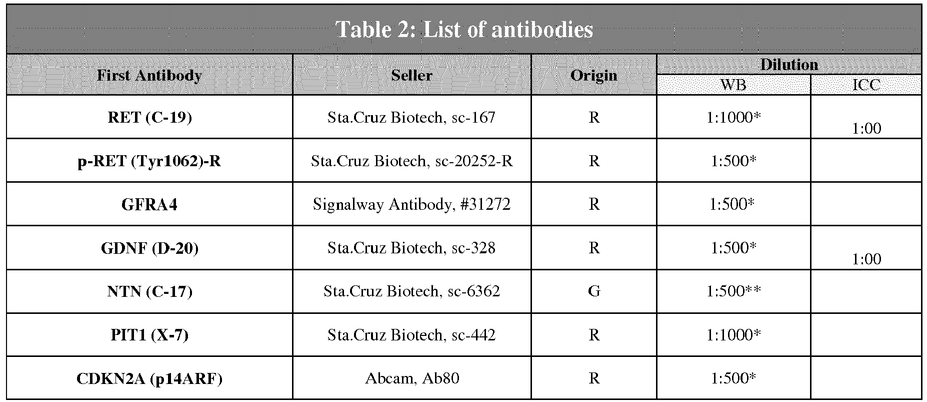

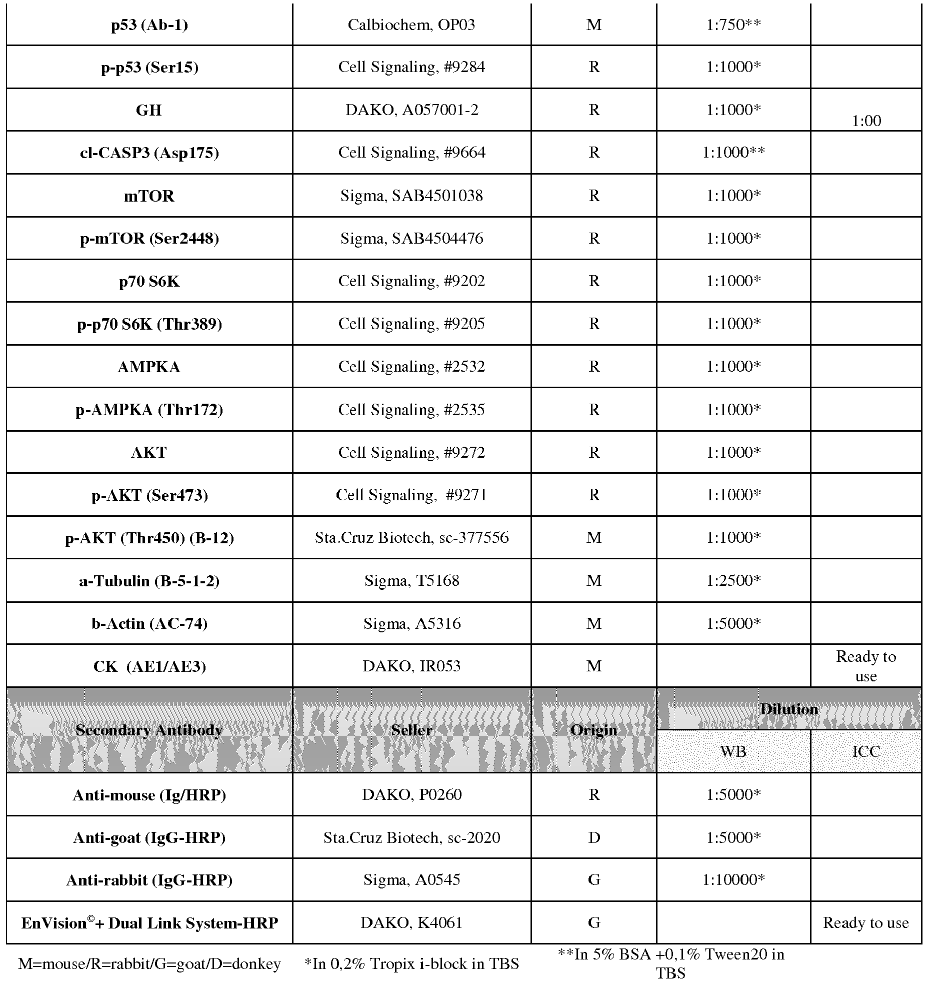

Enhanced chemiluminescence assay (Pierce ECL Western Blotting Substrate, 32106, Thermo Scientific, USA) was used for signal detection. Membrane was exposed to an X-ray film (4741 19289, Fujifilm, Japan) and developed using developer (Developer G150, AGFA HealthCare, Belgium) and fixator (Manual Fixing G354, AGFA HealthCare, Belgium).

### Analysis of secreted hGH and GDNF

10.000/well h7H-primary pituitary acromegaly (or NFPA) cells were seeded in a 96-well dish. Culture medium was collected and stored at -80°C for western blotting. For secreted hGH detection was performed by loading 15 microL in a western blot. Furthermore, 25 microL of culture medium were measured using COBAS hGH assay (Roche Diagnostics, Mannheim, Germany).

Secreted GDNF levels were measured with Human GDNF DuoSet ELISA (DY212, R&D Systems, USA) according to the manufacturer's protocol. Briefly, a 98-well microplate was coated with the capture antibody and incubated overnight at room temperature. Wells were blocked during 1 hour at room temperature and standards and culture medium samples were added and incubated 2 hours at room temperature. Detection antibody was added during 2 hours at room temperature. Then, streptavidin-HRP was added for 2 min avoiding direct light. After that, the substrate solution was added another 20 min avoiding direct light. The enzyme reaction was stopped by adding the stop solution. The plate was read at A450/A550nm using a microplate reader (Mithras LB 940, Berthold, Germany).

### Immunocytochemistry

Procedure was performed as described (Garcia-Rendueles A et al.: "Rewiring of the apoptotic TGF-beta-SMAD/NFkappaB pathway through an oncogenic function of p27 in human papillary thyroid cancer"; Oncogene 2017; 36:652-666). Briefly, h7H-primary pituitary acromegaly cells were seeded and grown on glass coverslips in a 48-well dish. Wells were washed and fixed with 10% neutral formalin for 30 minutes, and washed once followed by -20 °C methanol for 20 minutes at room temperature. After washing, permeabilization was performed with Triton 0·2% in H₂O on ice for one hour. Primary and Secondary Antibodies are listed in Table 2.

### Statistical analysis

Statistical analysis and plotting of graphs were performed using GraphPad Prism 6 (GraphPad Software, USA), SPSS Statistics 20 (IBM, USA) and Corel Draw Graphic Suite 2017. Analysis were performed in an independent way by CVA's group and the Mathematics Medical Department at CHUS. Graphs show mean±SEM. Normality for each group of quantitative data was assessed using the Kolmogorov-Sminorv test with Dallal-Wilkinson-Lillie for 'p' value. The majority of the groups were non-parametric. For statistical comparisons we performed detailed bi-variant analysis. As the initial step in analysis of the data, correlations were assessed with Spearman's Rho (rs) and significance. Quantitative variables were compared by Mann-Whitney tests when non-parametric or unpaired t-tests when parametric. Qualitative variables were compared with a Chi-square independence test, using the Pearson 'p' value when n>5 or the Fisher exact 'p' value when n<5. ANOVA was used for comparisons of more than two groups; for culture experiments, where there was one control with no treatment, Dunnet's correction was applied. For the multivariate analysis (response to first-line treatment and ARF expression cut-off depending on GNAS mutation) we used Chi-squared with multivariate contingency table analysis. To calculate the discriminating threshold for ARF (and GFRA4) expression, a ROC curve was used.

### B.RESULTS

We analysed quantitative RET pathway gene expression in a prospective series of 32 sporadic ACRO surgical samples collected in 2010-2017, in relation to clinical characteristics, and expression of known ACRO genes and the G-protein subunit α (GNAS) DNA mutation, a recurrent mutation found in sporadic ACRO.

In summary, 30 new patients and two undergoing reoperations were recruited from three hospitals (Santiago de Compostela, Madrid and Leon), comprising 66·6% females, median age 45·6 years old and median follow-up 4·2 years. 25% of samples contained mutated GNAS. The clinical characteristics of our series do not differ from other published series (Petrossians P et al.: "Acromegaly at diagnosis in 3173 patients from the Liege Acromegaly Survey (LAS) Database". Endocr Relat Cancer 2017; 24:505-518).

The majority of patients had a macroadenoma, with tumor volume and diameter being inversely related to age but positively related to hGH and IGF1 levels, Knosp grade and invasion. Presence of GNAS mutation was not related to any other variable. Success of surgery was correlated with smaller, less invasive tumors that secreted less hGH/IGF1. Resistance to adjuvant therapy with fgSSA was just significantly greater in males than females (p=0·049) but had no other significant correlation. An improved response to combined first-line treatment was observed in older patients (p=0·049) and smaller non-invasive tumors but had no other significant correlation.

### Example 1: Molecular taxonomy of the RET pathway in ACRO

Quantitative RNA expression analysis, using TaqMan qRT-PCR, was performed for the following gene groups: pituitary hormones (hGH isoforms; PRL; POMC/ACTH; CG-alpha, the common alpha subunit for FSH, LH and TSH; and the specific beta subunits of the two gonadotropins, FSHB and LHB), our pathways of interest (RET receptor as total expression RETN, long RETL and short RETS isoforms; its four co-receptors GFRA1-2-3-4 and its four ligands GDNF, NRTN, ARTM and PSPN; PIT1 transcription factor; p14ARF isoform from the CDKN2A gene (here called ARF); and p53), hypothalamic neuropeptide receptors (GHRHR and somatostatin receptors SSTR2, SSTR5), other pituitary transcription factors (PROP1, SF1, TPIT), AIP as a gene implicated in familial pituitary adenomas (FIPA), and two cell cycle genes previously studied in models of acromegaly (CDKN1A/p21 and CDKN1B/p27). We compared all genes with a commercial pool of pituitary poly-A mRNA as a technical control for each gene, to equalize measurement efficiency from different plates during the study.

First, we identified a common profile of genes enriched in ACRO, to allow us subsequently to evaluate individual samples. To assess enrichment, we compared the expression of each ACRO gene with a similar prospective series of Non-Functioning Pituitary Adenomas (NFPA, n=63). As expected, hGH (22KDa (hGHTaq) and 20 KDa (GHSyb) isoforms), GHRHR, SSTR2 and SSTR5 were very abundant in ACRO in comparison with NFPA (**Figure 1A**).

There was no difference between ACRO and NFPA in expression levels of other non-somatotroph hormones, such as POMC (corticotroph hormone), CG-alpha or LHB (the beta subunit of the LH, one of the two gonadotroph hormones). However, FSHB (the beta subunit of FSH, the other gonadotroph hormone) was significantly enriched in NFPA.

From the RET receptor downstream, all genes in the RET pathway were significantly more highly expressed in ACRO than in NFPA (**Figure 1A**)**.** Especially abundant were the ligand GDNF, the somatotroph transcription factor PIT1 and the tumor suppressor ARF, while a smaller difference was found in p53, attributable to its regulation being at the protein level. Of the three non-somatotroph transcription factors studied, PROP1 presented low expression in all tumors, although it was significantly higher in ACRO than in NFPA; as expected, SF1 was significantly higher in NFPA, while TPIT was poorly expressed in both ACRO and NFPA, attributable to the fact that it is characteristic of corticotroph tumors (ACTH series) (**Figure 1A**). AIP, a protein related to familial but not sporadic acromegaly, did not show differences in expression between ACRO and NFPA (**Figure 1A**). p21, a gene activated by p53, was more abundant in ACRO (**Figure 1A**), contrary to p27 that did not present differences.

After this initial characterization of the ACRO series, each sample was individually evaluated for its level of enrichment of the most abundant somatotroph genes (GH, GHRH, PIT1) and depletion of non-somatotroph genes (TPIT, POMC, SF1, CG-alpha, FSHB, LHB). PRL was considered a 'neutral' gene as it can be co-secreted with GH. All samples passed our technical control, although two samples, ACRO23 - a reoperation following radiotherapy - and ACRO39, expressed some non-ACRO genes. However, the level of hGH expression in these two samples was still much higher than that in NFPA and thus they were included. PRL was co-expressed with hGH in three samples: ACRO19, 25 and 32. We proceeded with our study in the confidence that the tissue from which we had extracted RNA was mainly ACRO.

**Figure 1B** shows the RET pathway in normal somatotroph cells (Canibano C et al.: "The dependence receptor Ret induces apoptosis in somatotrophs through a Pit-1/p53 pathway, preventing tumor growth"; EMBO J 2007; 26:2015-2028 and Diaz-Rodriguez E et al.: "Direct promoter induction of p19Arf by Pit-1 explains the dependence receptor RET/Pit-1/p53-induced apoptosis in the pituitary somatotroph cells. Oncogene 2012; 31:2824-2835).

In the absence of the ligand GDNF, RET is a dependence receptor: the intracellular region is processed by Caspase-3, generating an intracellular fragment IC-RET and activation of PKC delta (PKCd); this induces PIT1 overexpression, exceeding permissive levels of an already abundant gene and inducing ARF expression. ARF binds to and blocks MDM2, the protein that ubiquitinates p53 prior to its destruction, leading to p53 accumulation and apoptosis. If the ligand is present, GDNF induces RET dimerization which inhibits the apoptotic pathway, activating AKT and maintaining regulated PIT1 expression, which is sufficient to produce hGH but not to induce apoptosis. This pathway explains the abundance of GDNF both in the normal pituitary (containing 50-60% of somatotroph cells) and to a greater extent in ACRO (**Figure 1A**).

RET has a long (RETL) and a short (RETS) isoform varying in the length of its cytoplasmatic C-terminal tail, leading to different number of tyrosines candidates for phosphorylation and hence interaction with different substrates (**Figure 1B****,** right). As stated above, each RET co-receptor binds a characteristic ligand (GDNF-GFRA1, NRTN-GFRA2, ARTM-GFRA3, PSPN-GFRA4). (see STRING, https://string-db.org/cgi/network.pl?taskld=Unm9pNIMfJex). It is well established that GDNF activates RET through GFRA1 (Jing S, et al.: "GDNF-induced activation of the ret protein tyrosine kinase is mediated by GDNFR-alpha, a novel receptor for GDNF"; Cell 1996; 85:1113-1124; Treanor JJ et al.: "Characterization of a multicomponent receptor for GDNF"; Nature 1996; 382:80-83). It has also been demonstrated that NRTN is able to activate RET through GFRA2 more potently than GDNF. However, there is also cross-activation between ligands and co-receptors, as shown by transfection experiments and in primary culture. Thus NRTN is able to fully activate RET with equal potency to GDNF through GFRA1 in transfection studies although it does so slightly less potently than GDNF in primary cells expressing GFRA1 receptor (**Figure 1B**). ARTM and PSP are less potent than GDNF or NRTN but still able to activate RET through GFRA1 in cells expressing the receptor (Baloh RH et al. "TrnR2, a novel receptor that mediates neurturin and GDNF signaling through Ret"; Neuron 1997; 18:793-802; Jing Set al.: "GFRalpha-2 and GFRalpha-3 are two new receptors for ligands of the GDNF family"; J Biol Chem 1997; 272:33111-33117; Baloh RH et al.: "Artemin, a Novel Member of the GDNF Ligand Family, Supports Peripheral and Central Neurons and Signals through the GFRα3-RET Receptor Complex". Neuron 1998; 21:1291-1302; Milbrandt J et al.: "Persephin, a novel neurotrophic factor related to GDNF and neurturin"; Neuron 1998; 20:245-253; Sidorova YA et al: "Persephin signaling through GFRalpha1: the potential for the treatment of Parkinson's disease" Mol Cell Neurosci 2010; 44:223-232).

In the ACRO series studied, expression of RETL and RETS isoforms were similar as approximate halves of total RET expression (RETN) (**Figure 1C**). The co-receptor most abundantly expressed was GFRA1 followed by GFRA4 (**Figure 1C**). This fitted with the major ligand expressed being GDNF, followed by NRTN and PSPN (**Figure 1D**).

We calculated correlations between molecular variables. Spearman correlations were used to investigate gene expression and GNAS mutation. Significant but relevant correlations (Rho, ARTM ligands, and both RET isoforms). Both GDNF and NRTN were negatively correlated with PIT1 but positively correlated with PROP1. PIT1 and PROP1 were negatively correlated with each other. PROP1 directly induces PIT1 expression during embryonic development, and once PIT1 is induced PROP1 is repressed through a mechanism that is known not to be directly repressed by PIT1 (Cushman LJ yet al.: "Persistent Prop1 expression delays gonadotrope differentiation and enhances pituitary tumor susceptibility"; Hum Mol Genet 2001; 10:1141-1153). GFRA1 and SF1 were also positively correlated with PROP1. ARF expression was positively correlated with GH, GHRHR, AIP, ARTN and TPIT. SSTR2 was only correlated with GH expression. No correlations were found for p53, which was not unexpected as p53 is regulated mainly at the protein level. All other genes studied (including p21 and p27) and GNAS mutations were found not to be relevantly correlated.

The most relevant finding from these data was that the GDNF-RET survival pathway is strongly expressed in acromegaly and reinforced by redundancy in expression of RET ligands and RET receptor isoforms, controlling PIT1 and inhibiting the RET apoptotic pathway. This prompted us to further explore the RET pathway in relation to clinical follow-up characteristics of the patients.

### Example 2: ARF is a prognostic factor of response to first-line combined treatment (surgery+fgSSA) in acromegaly

We investigated correlations between molecular and clinical variables, which were divided into three groups: diagnostic, pathologic and prognostic/follow-up. In **Figure 2A** significant and relevant (rs≥0·5) correlations are shown.

Of the analytical diagnostic variables, only basal serum GH at diagnosis was significantly correlated with GH, SSTR2, AIP and p53 RNA tumor expression, all of which were abundantly expressed in ACRO (**Figure 2A** left).

Regarding pathologic variables, two genes showed important correlations, ARF and TPIT. ARF expression was negatively correlated with invasiveness, Knosp grade and, importantly, with post-surgical residual tumor (**Figure 2A** center). TPIT expression was negatively correlated with tumor diameter/volume, invasiveness and Knosp grade, but positively correlated with surgical cure (**Figure 2A** right).

In the group of prognostic variables, response to fgSSA during follow-up was strongly and positively correlated with ARF expression (rs>0·8), while GFRA4 was negatively correlated (rs>0·7). Other genes less strongly correlated with response to fgSSA were GH, TPIT and SSTR2 (rs 0·5-0·6). Since expression of the gene with the two most important correlations, ARF, was also related to a favorable course of disease (negatively correlated with residual tumor and positively correlated with response to fgSSA), we combined the two prognostic variables into a single 'Good Prognosis' Group 1, defined as responsive to combined first-line therapy (surgery + fgSSA). When this group was analysed for correlations, ARF expression showed the highest positivity (rs>0·7) and significance, followed by TPIT (rs=0·6), while GFRA4 was negatively correlated (rs=0·5).

We then compared Group 1 with 'Resistant' Group 0, defined as not cured by surgery and resistant to fgSSA during follow-up. ARF mRNA expression values were much lower in Group 0 than Group 1 (p<0·0001, **Figure 2C** left). Similar results were obtained when tumors not cured by surgery were analysed separately. ARF was again more highly expressed in samples responding to fgSSA than resistant samples (**Figure 2C** right). Differences between follow-up groups for the other genes tested (SSTR2, SSTR5, TPIT, SF1) either did not reach the ARF levels of significance or were not significant (**Figure 3**). Moreover, results did not change for ARF when ACTB was used as the control gene, instead of TBP to normalize RNA expression (**Figure 3**).

We then aimed to find a cut-off value for ARF expression which would classify an acromegaly as either Responsive Group 1 or Resistant Group 0 at the time of surgery, by constructing a receiving operating curve (ROC) of ARF levels (**Figure 3D**). We found that placing the cutoff at ≥0·06 separated the groups with 100% sensitivity but 85·7% specificity. Placing the cutoff at ≥0·1, however, separated groups with 96% of sensitivity and 100% specificity. Although these values were very good as a clinical predictor we wanted to understand if there were other factors that might explain the blurred separation of three samples located at cut-off levels. In Group 1 the only value in the range 0·06-0·1 was in fact GNAS-mutated, while in Group 0 values in the same range were non-GNAS-mutated. We did not find any correlations between GNAS mutation and any molecular or clinical variables studied. Moreover, while the median values were no different between the GNAS-mutated and non-mutated groups, the range of ARF expression in the mutated group was much smaller (range 0·042-0·64, n=8) than that in the non-mutated group (range 0·003-1·08, n=24) (**Figure 3E**). Therefore, a lower cut-off value would be expected for the mutated GNAS samples. In accordance with this, when we separated Group 0 and Group 1 using a cutoff of 0·1 for ARF expression in non-mutated GNAS ACRO and 0·06 in mutated GNAS ACRO, and performed a multivariant analysis using Chi-square test, we found maximal significance (p<0·0000) (**Figure 2D** top), with all samples showing 100% sensitivity and specificity (**Figure 2D** lower).

fgSSA include two prescriptions, Octeotride LAR (long acting release) (Octeotride, n=5 patients) and Lanreotide Autogel (Lanreotide, n=13 patients). We separated patients receiving each treatment and found that, again, ARF expression was able to separate Group1 from Group 0 for both treatments (**Figure 3F**). We performed statistics in the Lanreotide group of patients, and again differences in ARF levels were clearly significant between Group 0 (Resistant) and Group 1 (Responsive), in spite of their reduced number.

GFRA4 was the only negatively correlated gene during follow-up (**Figure 2B**). When all samples were taken into account there were significant higher expression levels in Group 0 than Group 1, although the gene was not a perfect classifier (**Figure 2E** left). Selecting samples that were not cured with surgery and treatment with fgSSA (Resistant Group 0, Responsive Group 1), GFRA4 expression was again significantly higher in Group 0, with a cut-off ≥0·1 (**Figure 2E** right). There was a single sample in Group 0 that did not follow this rule (yellow dot=ACRO36), which was from a reoperation following radiotherapy in the same patient as ACRO27. This could be because radiotherapy or fgSSA affected expression of GFRA4, making its value as a prognostic marker for response to fgSSA limited to adenomas not cured by a first operation.

To test whether the results obtained for gene expression were verifiable with protein expression we studied relevant protein expression in a sub-set of samples (**Figure 2F**). We compared one resistant sample (ACRO27) with five responsive, either cured by surgery (ACRO7, 8) or responsive to fgSSA therapy (ACRO9, 16 and 15). Only ACRO8 and ACRO27 were naive samples that had not been pre-surgically treated with fgSSA, at least for a short time while waiting for the surgery. As shown in **Figure 2F****,** ACRO27 contained high levels of RET protein but negligible or low levels of PIT1, ARF and p53. Only ACRO7, 8 and 27 had detectable GFRA4 expression, but the proteins detected were of three different molecular weights. It has previously been shown that GFRA4 has a canonical protein GPI isoform that makes a GPI link on the extracellular aspect of the plasma membrane and functions as a RET co-receptor, as well as a longer TM isoform with a transmembrane domain, and a shorter secreted isoform (Lindahl M et al.: "Expression and alternative splicing of mouse Gfra4 suggest roles in endocrine cell development"; Mol Cell Neurosci 2000; 15:522-533). Thus we could ascertain that ACRO7 expressed the secreted GFRA4 isoform but ACRO8 the TM isoform. Only ACRO27 presented the canonical GPI isoform able to function as a RET co-receptor.

### Example 3: Tyrosine Kinase Inhibition as potential therapy for SSA Resistant Acromegaly

Taken together, the data of previous examples indicated that the RET/PIT1/ARF/p53 apoptosis pathway is maintained in ACRO that are responsive to first-line therapy, but blocked and re-oriented towards survival in tumors that are resistant to first-line therapy. To counteract apoptosis, RET must bind GDNF, the main ligand in ACRO, dimerize and activate survival pathways through its tyrosine kinase (TK) activity. We therefore explored the possibility that any of the TK inhibitors (TKI) currently used to treat neuroendocrine tumors might be able to block the RET/GDNF survival pathway.

To do so we needed to develop a primary cell culture system that would allow repeated experiments and dose-response analyses, while maintaining characteristics relevant to the disease. Previously, we and others have performed acute experiments with cell dispersions of human ACRO or rat pituitaries in culture conditions that do not allow cell growth or proliferation (see material and methods, section "h7H cell culture"; Garcia A et al.: "Regulation of Pit-1 expression by ghrelin and GHRP-6 through the GH secretagogue receptor"; Mol Endocrinol 2001; 15:1484-1495). The few pituitary cell lines with a somatotroph phenotype are rat, and not human, and do not maintain the full secretory phenotype, expressing Gh and Pit1 but lacking essential receptors like Ghrhr or very reduced Ret expression.

In 2012, we established new culture conditions for human thyroid, h7H, in which all components were adapted to physiological concentrations found in human serum (Bravo SB et al.: "Humanized medium (h7H) allows long-term primary follicular thyroid cultures from human normal thyroid, benign neoplasm, and cancer" J Clin Endocrinol Metab 2013; 98:2431-2441). Through culturing normal and neoplastic follicular cells, h7H has led to the discovery of specific mechanisms underlying human thyroid cancer. We adapted h7H medium for growing human pituitary adenomas by varying the hormone mixture (see Material and Methods). We successfully (about 75% success) grew 8 NFPA and 3 ACRO which maintained their phenotype for at least twenty-five passages (indicated as P-NFPA or P-ACRO, **Figure 4A**). Cultures showed the same genetic identity profiles to the original pituitary tissue, which was distinct from any known human cell line. Cells grew slowly (with a passage 1:2 every two weeks) and were positive for cytokeratins, GH, RET and GDNF assayed immunocytochemically. Human GH was detected by western blot in the culture medium of the three P-ACRO only after incubation, showing that it was being secreted during culture. We also measured hGH in the culture medium, with a standard kit to measure hGH in plasma, following multiple passages of the cell cultures, until cell stop growing. We found that they secreted hGH in high quantities, confirming the western blot experiments (**Figure 4B**)**.** Since GDNF was the main RET ligand expressed in ACRO (**Figure 1A**), we also measured its secretion into the culture medium. **Figure 4C** shows the concentration of human GDNF (hGDNF) secreted by the three P-ACRO, while no hGDNF was detected in either P-NFPA or medium cultured in empty wells. In P-ACRO32 medium GDNF concentration increased as the culture grew.

We selected five TKI, Vandetanib (V), Lenvatinib (L), Sunitinib (Su), Cabozantinib (C) and Sorafenib (So), all FDA approved for treatment of neuroendocrine cancers and known to inhibit RET kinase. A key step in our assay was to decide which TKI concentrations tested in vitro were relevant to their in vivo actions. We therefore performed an in silico study to ascertain relevant mean concentrations at the steady-state (Cmₛₛ), as detected in sera from patients treated with the TKIs in Phase II/III clinical trials. Sorafenib was used at half this concentration since 10-20% of the patients required their dose to be halved due to serious side effects.

As shown in **Figure 4D****,** the three cultured P-ACRO maintained the RET apoptotic pathway and presented marked apoptosis when deprived of GDNF (by washing and replacing with low-serum medium) that was inhibited by re-addition of GDNF (100 or 500 ng/ml). In the absence of GDNF different TKIs affected RET-dependent apoptosis to different extents. Only Sorafenib caused significant and appropriate apoptosis in the presence of GDNF, neutralizing the survival effect of both 100 and 500 ng/ml GDNF in the three cultures (**Figure 4D**). We performed a dose-response curve of Sorafenib in two of the cultures. In both P-ACRO30 and P-ACRO32 2 and 4 µg/ml led to significant inhibition of the GDNF survival effect (**Figure 4E**), suggesting that lower doses than those required in cancer could be effective in acromegaly treatment. Some ACRO showed a combined expression of GDNF and NRTN, the second most important ligand for RET (**Figures 1B** and **2F**) which can cross-activate RET through GFRA1. In accordance with this, in our culture system NRTN alone or combined with GDNF was also a survival factor following ligand deprivation (**Figure 4F**). We therefore tested whether Sorafenib was able to counteract the survival effect of GDNF or NRTN or the combination of both, and indeed found that it could neutralize the survival action of each factor alone or in combination (**Figure 4F**).

ACRO tumors expressed both isoforms of the RET receptor, RETL and RETS (**Figure 1C** and **2F**), which have distinct thyrosines and differentially activating signal transduction pathways, either quantitatively (intensity, duration) or qualitatively (activated signaling cascades) (Mulligan LM: "RET revisited: expanding the oncogenic portfolio"; Nat Rev Cancer 2014; 14: 173-186). P-ACRO maintained expression of both RET isoforms in culture (**Figure 4A**). We investigated the anti-survival action of Sorafenib on each RET isoform by using either RETS or RETL transfection into the rat pituitary cell line GH4C1 that expresses GH, PIT1 and GDNF, but RET at very low levels. In this set-up, Sorafenib was able to block the survival action of rat GDNF (rGDNF) in the presence of either human isoform RETL or RETS, much more effectively than any of the other TKIs tested (**Figure 4G**). The other TKIs (V, L, Su, C) were able to counteract GDNF survival, but also affected the RET apoptotic pathway in the absence of ligand (**Figure 4G**).

We next investigated which signal transduction pathways activated by RET/GDNF were being affected by Sorafenib. AKT has been implicated in GDNF/RET survival in normal somatotrophs and acromegaly (Canibano C et al.: "The dependence receptor Ret induces apoptosis in somatotrophs through a Pit-1/p53 pathway, preventing tumor growth"; EMBO J 2007; 26:2015-2028; Diaz-Rodriguez E et al.: "Somatotropinomas, but not Nonfunctioning Pituitary Adenomas, maintain a Functional Apoptotic RET/Pit1/ARF/p53 Pathway that is Blocked by excess GDNF" Endocrinology 2014; 155:4329-4340).

We therefore performed an antibody array with extracts of two primary acromegaly cultures, P-ACRO30 and P-ACRO32, treated for one hour with/without GDNF plus/minus Sorafenib (**Figure 5A**). Arrays were scanned and quantified (**Figure 5B**). Kinases significantly activated by GDNF were AKT and mTOR. Significantly inhibited by GDNF were the cleavage of Caspase-3 (cl-CASP3) and PARP (cl-PARP), two enzymes activated by cleavage, as well as Ser15 phosphorylation of p53 (p-p53) a site well-known for p53 stability and activity, all of which are implicated in apoptosis in somatotrophs. Sorafenib blocked GDNF-induced AKT/mTOR activation and increased cl-Caspase-3 and cl-PARP and p-p53. Moreover, Sorafenib caused activation of p-AMPKa per se, which was augmented in the presence of GDNF (**Figure 5B**).

To validate the array results we performed western blots with extracts from P-ACRO32, also treated with GDNF/Sorafenib for one hour. Lysates were obtained with buffers optimised for enrichment in the plasma membrane or cytoplasm. In parallel, whole cell extracts were treated for 24 hours, the time at which apoptosis or survival was clearly detectable (see **Figure 4**). As expected, GDNF induced RET phosphorylation at the membrane (**Figure 5C**). Sorafenib blocked this phosphorylation and even reduced total RET expression. Previous studies have demonstrated that in the absence of GDNF the RET apoptotic pathway is initially activated after a complex between RET and Caspase-3 is formed at the membrane, resulting in both RET processing and Caspase-3 activation by cleavage (IC-RET and cl-CASP3 fragments, respectively); this also occurred in our experiment (**Figure 5C** and **D**). When the cells are deprived of serum/GDNF cl-CASP3 increases but this was blocked in the presence of GDNF as described. GDNF in the presence of Sorafenib recovered and even enhanced cl-Casp3 levels (**Figure 5C**).

At the cytoplasmic level (**Figure 5D**), GDNF induced phosphorylation of AKT at (mainly) Thr450 and Ser473, which was blocked by Sorafenib. As a corollary, GDNF-induced mTOR phosphorylation was also blocked by Sorafenib, as was the mTOR-dependent phosphorylation of S6 kinase (**Figure 5D**). Deprivation induced RET processing with appearance of the cytoplasmic Caspase-3 processed IC-RET fragment; RET processing is inhibited by GDNF but recovered by Sorafenib (**Figure 5D**). On the other hand, Sorafenib alone induced AMPKa activating phosphorylation; this activation was enhanced in the presence of GDNF. Similarly, Sorafenib induced the activating phosphorylation of p53 that was enhanced -and not inhibited- in the presence of GDNF (**Figure 5D**).

After 24 hours of treatment (**Figure 5E**), PIT1 accumulated in serum-deprived cells, leading to an increase in p14ARF. GDNF reduced both PIT1 and ARF levels, and this was prevented in the presence of Sorafenib. In parallel, serum deprivation induced p53 accumulation which was blocked by GDNF. Sorafenib led to recovery of p53 protein levels and to apoptosis (**Figure 4**).

### C. DISCUSSION

The data from this molecular study of sporadic acromegaly tumors demonstrate that expression of elements of the RET pathway are hallmarks of acromegaly. GDNF, in particular, is strongly enriched in ACRO compared with normal pituitary and NFPA. The two RET isoforms RETL and RETS are equally expressed within ACRO. GFRA1 is the main co-receptor expressed in ACRO, followed by GFRA4. NRTN is also expressed in some ACRO. Correlations among expression of various ligands and RET indicate redundancy of the RET survival pathway that completely inhibits the RET apoptotic pathway.

The most striking result of our work is that ARF mRNA expression is a sensitive and specific marker of good prognosis. High ARF levels guarantee response while low ARF predict resistance to first-line therapy (surgery plus adjuvant therapy with fgSSA). The confidence level reaches 100% if GNAS mutation is used to slightly adjust the cut-off (≥0·1 for non-mutated GNAS and ≥0·06 for mutated GNAS). This cut-off was initially obtained using TBP as the control gene to adjust for overall expression, and could also be obtained using another control gene such as ACTB. We also obtained results at the protein level in tissues from tumors that fully corroborated the mRNA level data.

mRNA expression is directly related to protein expression for ARF since it is an mRNA isoform of the CDKN2A gene, that also encodes p16INK4. The two isoforms have different promoters and exon 1; exons 2 and 3 are shared but with different reading frames of translation, giving rise to two different proteins. Initial studies proposed ARF as a marker of good prognosis in cancer, although this idea was subsequently discarded as aggressive cancers either mutate p53, becoming independent of ARF, or methylate the ARF promoter (Kotsinas Aet al: "ARF: a versatile DNA damage response ally at the crossroads of development and tumorigenesis"; Front Genet 2014; 5:236). However, whole genome sequencing has repeatedly shown, that somatotropinomas are benign adenomas with non-mutated p53 (as well as RET and ARF). Moreover, mouse and cell culture models have demonstrated an essential role for ARF and p53 in the regulation of pituitary cellularity. Furthermore, inhibitors of the interaction between MDM2 and p53, which degrades p53, induces senescence in pituitary adenomas.

Current acromegaly treatment takes a trial-and-error approach: first surgery is followed by evaluation at 4-16 weeks, and a trial with fgSSA is evaluated after 6-12 months. After that, 20-25% of patients (as in our series) undergo expensive second-line SSA therapy to which they are also frequently resistant while the peripheral disease continues unabated due to high GH/IGF1 levels and continued tumor growth (Puig Domingo M: "Treatment of acromegaly in the era of personalized and predictive medicine"; Clin Endocrinol (Oxf) 2015; 83:3-14; Kasuki L et al.: "Management of Endocrine Disease: personalized medicine in the treatment of acromegaly"; Eur J Endocrinol 2018; 178:R89-R100).

Risk factors related to a poor prognosis are age and male sex, size and volume of the adenoma, Knosp grade and extent of invasion. However, these risk factors are more related to whether the surgery is curative without causing hypopituitarism than to the biology of the tumor per se: a larger tumor impinges on and invades surrounding structures, making surgery less likely to be successful. Importantly, however, not all the macroadenomas or invasive tumors are resistant to therapy. Thus, none of these factors, nor the combination of them, can predict with precision the response to adjuvant fgSSA therapy or to combined first-line treatment.

Several molecular factors have been proposed for early identification, at the time of initial surgery, of SSA-resistant tumors. SSTR2 receptor expression has been the most widely studied, using immunohistochemistry and mRNA expression, as it mediates SSA action on somatotroph cells. SSTR2 protein expression is correlated with a response to SSA. However, while the predictive negative value is remarkable, there is no clear cut-off for SSTR2 expression levels to separate resistant from responsive tumors, both because levels can be affected by pre-surgical treatment and resistance might be mediated by a post-receptor mechanism. This is also seen in our current series in relation to SSTR2 expression which does not discriminate between Responsive and Resistant'. Although tumor granulation (positiveness for CAM5.2) is also related to increased expression of SSTR2 pre-surgical treatment and other factors affect its immunostaining too much for it to be a clear marker.

Other proposed molecular markers include SSTR5, the other SSA receptor expressed in somatotrophs, dopamine receptors or a combination of the two, and non-receptor proteins such as ZAC1, E-cadherin, B-arrestin, AIP and Ki67. However, none of these have reached the consistency or sensitivity of a reliable prognostic marker. The examples here clearly show that ARF is useful as a marker.

The early identification of SSA-resistant patients will also inform the search for other anti-ACRO pharmacological therapies. In the current study, we have explored the possibility that TKIs, already used to treat endocrine cancers, might have an effect against the RET/GDNF/survival pathway and could be useful in acromegaly. To that end, we have developed a new culture system, adapted from a previous study of human thyroid tumors that has enabled us to amplify the number of adenoma cells and perform functional and signaling experiments in ACRO cultures while maintaining the cellular phenotype. Importantly, TKIs were tested at relevant concentrations with respect to serum levels in patients. Of the TKIs tested, only Sorafenib was able to inhibit the survival action of GDNF through RET tyrosine kinase activation without affecting the normal RET/PIT1/ARF/p53 apoptotic pathway in the absence of GDNF. Lenvatibib, Cabozantinib, Sunitinib and Vandetanib seemed to affect both apoptosis and survival pathways, and thus to have no net effect. It could be that these four TKIs are affecting kinases required by the RET/apoptotic pathway, such as PKCdelta or JNK, which phosphorylates and recruits CREB and c/EBPa, permanently activating the PIT1 promoter and leading to apoptosis (Canibano C et al.: "The dependence receptor Ret induces apoptosis in somatotrophs through a Pit-1/p53 pathway, preventing tumor growth"; EMBO J 2007; 26:2015-2028).

Sorafenib, on the other hand, had a dose-response effect and seemed sufficiently potent against GDNF to function at lower doses than those required in cancer therapy. In our in vitro studies, we have used half the concentration of that found in serum of the patients in Phase III studies since a relevant percentage of patients needed a dose reduction due to adverse effects. Moreover, the dose-response curve indicated that even lower doses were effective. In any case, if the treatment were effective for acromegaly, inducing apoptosis, it may be expected a limited treatment for a period of time until resumption or reduction of the adenoma.

As previously mentioned, NRTN is also able to activate RET through the GFRA1 co-receptor, and some ACRO also express NRTN. However, Sorafenib not only blocks the survival action of GDNF but also that of NRTN and the combination GDNF+NRTN present in some tumors. Finally, both RET isoforms, RET-L and RET-S, were expressed in both ACRO tumors and cultures. Sorafenib was also able to block survival without affecting apoptosis in rat pituitary cells transfected specifically with RETL or RETS.

At the molecular level, Sorafenib was able to directly (after a one-hour treatment) inhibit GDNF-induced RET phosphorylation, promoting RET degradation. As a consequence, GDNF-induced AKT and mTOR phosphorylation were also blocked by Sorafenib. Sorafenib activated AMPKa kinase, which has previously been demonstrated to be by the direct action of Sorafenib on mitochondria by which it reduces the ATP/AMP quotient (Ross FA et al: "Mechanisms of Paradoxical Activation of AMPK by the Kinase Inhibitors SU6656 and Sorafenib"; Cell Chem Biol 2017; 24:813-824.e4). However, in the ACRO cultures Sorafenib AMPKa activation was potentiated in the presence of GDNF, suggesting that the mTOR blockade might be involved. AMPKa activation has previously been proposed as essential for Sorafenib activity. AMPKa activation is also implicated in cellular autophagy, although it is not clear whether Sorafenib's anti-tumor activity is necessarily mediated by autophagy. The possibility remains that other AKT/mTOR related survival pathways inhibited by Sorafenib could play important roles. The overall consequence of AMPKa activation or AKT/mTOR inhibition or both was Caspase-3 activation with RET processing obtaining the cytoplasmic IC-RET fragment, an accumulation of PIT1, ARF and p53, and apoptosis, despite the presence of GDNF.

Other data that could be of interest in the future included expression of PROP1 in ACRO and the role of the GFRA4 co-receptor. PROP1 is a transcription factor characteristic of embryonic pituitary progenitors and maintained in postnatal pituitary stem cells. During differentiation, PROP1 activates PIT1 while itself being repressed. In ACRO, GDNF was negatively correlated with PIT1, but positively with PROP1 expression, suggesting a role for GDNF in inducing a more progenitor-like/less differentiated phenotype in adenoma somatotroph cells. Previous studies in the mouse have demonstrated that three different Gfra4 isoforms expressing GPI, soluble or transmembrane proteins are present in the thyroid and pituitary. Only the Gfra4 GPI and soluble isoforms, and not the transmembrane isoform, are able to act as RET co-receptors. Our results in ACRO, measuring total GFRA4 mRNA and detecting specific protein isoforms, suggest that the GFRA4 GPI isoform specifically is associated with resistance to adjuvant SSA therapy in acromegaly.

In summary, we propose that tumor ARF mRNA expression measured at the time of the surgery is a prognosis factor in acromegaly, and that a new pharmacotherapy with Sorafenib could be effective. Ultimately, the ability to identify patients who are resistant to first-line therapy and the trial of new pharmaceuticals should improve outcomes in acromegaly.

### References:

1 Ezzat S, Asa SL, Couldwell WT, Barr CE, Dodge WE, Vance ML, McCutcheon IE. The prevalence of pituitary adenomas: a systematic review. Cancer 2004; 101:613-619.
2 Hernández-Ramírez LC, Gabrovska P, Dénes J, Stals K, Trivellin G, Tilley D, Ferrau F, Evanson J, Ellard S, Grossman AB, Roncaroli F, Gadelha MR, Korbonits M; International FIPA Consortium.. Landscape of Familial Isolated and Young-Onset Pituitary Adenomas: Prospective Diagnosis in AIP Mutation Carriers. J Clin Endocrinol Metab 2015; 100: E1242-1254.
3 Pivonello R, Auriemma RS, Grasso LF, Pivonello C, Simeoli C, Patalano R, Galdiero M, Colao A. Complications of acromegaly: cardiovascular, respiratory and metabolic comorbidities. Pituitary 2017; 20:46-62.
4 Terzolo M, Reimondo G, Berchialla P, Ferrante E, Malchiodi E, De Marinis L, Pivonello R, Grottoli S, Losa M, Cannavo S, Ferone D, Montini M, Bondanelli M, De Menis E, Martini C, Puxeddu E, Velardo A, Peri A, Faustini-Fustini M, Tita P, Pigliaru F, Peraga G, Borretta G, Scaroni C, Bazzoni N, Bianchi A, Berton A, Serban AL, Baldelli R, Fatti LM, Colao A, Arosio M; Italian Study Group of Acromegaly. Acromegaly is associated with increased cancer risk: a survey in Italy. Endocr Relat Cancer 2017; 24: 495-504.
5 Gadelha MR, Kasuki L, Lim DS, Fleseriu M. Systemic complications of acromegaly and the impact of the current treatment landscape: an update. Endocr Rev 2018; doi: 10.1210/er.2018-00115.
6 Melmed S, Bronstein MD, Chanson P, Klibanski A, Casanueva FF, Wass JAH, Strasburger CJ, Luger A, Clemmons DR, Giustina A. A Consensus Statement on acromegaly therapeutic outcomes. Nat Rev Endocrinol 2018; 14: 552-561.
7 Giustina A, Chanson P, Kleinberg D, Bronstein MD, Clemmons DR, Klibanski A, van der Lely AJ, Strasburger CJ, Lamberts SW, Ho KK, Casanueva FF, Melmed S; Acromegaly Consensus Group. Expert consensus document: A consensus on the medical treatment of acromegaly. Nat Rev Endocrinol 2014; 10: 243-248.
8 Colao A, Auriemma RS, Lombardi G, Pivonello R. Resistance to somatostatin analogs in acromegaly. Endocr Rev 2011; 32: 247-271.
9 Garcia-Lavandeira M, Diaz-Rodriguez E, Garcia-Rendueles ME, Rodrigues JS, Perez-Romero S, Bravo SB, Alvarez CV. Functional role of the RET dependence receptor, GFRa co-receptors and ligands in the pituitary. Front Horm Res 2010; 38: 127-138.
10 Mulligan LM. RET revisited: expanding the oncogenic portfolio. Nat Rev Cancer 2014; 14: 173-186.
11 Ibanez CF, Andressoo JO. Biology of GDNF and its receptors - Relevance for disorders of the central nervous system. Neurobiol Dis 2017; 97:80-89.
12 Urbano AG, Suarez-Penaranda JM, Dieguez C, Alvarez CV. GDNF and RET-gene expression in anterior pituitary-cell types. Endocrinology 2000; 141:1893-1896.
13 Japon MA, Urbano AG, Saez C, Segura DI, Cerro AL, Dieguez C, Alvarez CV. Glial-derived neurotropic factor and RET gene expression in normal human anterior pituitary cell types and in pituitary tumors. J Clin Endocrinol Metab 2002; 87:1879-1884.
14 Negulescu AM, Mehlen P. Dependence receptors - the dark side awakens. FEBS J 2018; 285:3909-3924.
15 Canibano C, Rodriguez NL, Saez C, Tovar S, Garcia-Lavandeira M, Borrello MG, Vidal A, Costantini F, Japon M, Dieguez C, Alvarez CV. The dependence receptor Ret induces apoptosis in somatotrophs through a Pit-1/p53 pathway, preventing tumor growth. EMBO J 2007; 26:2015-2028.
16 Diaz-Rodriguez E, Garcia-Lavandeira M, Perez-Romero S, Senra A, Cañibano C, Palmero I, Borrello MG, Dieguez C, Alvarez CV. Direct promoter induction of p19Arf by Pit-1 explains the dependence receptor RET/Pit-1/p53-induced apoptosis in the pituitary somatotroph cells. Oncogene 2012; 31:2824-2835.
17 Diaz-Rodriguez E, Garcia-Rendueles AR, Ibanez-Costa A, Gutierrez-Pascual E, Garcia-Lavandeira M, Leal A, Japon MA, Soto A, Venegas E, Tinahones FJ, Garcia-Arnes JA, Benito P, Angeles Galvez M, Jimenez-Reina L, Bernabeu I, Dieguez C, Luque RM, Castaño JP, Alvarez CV. Somatotropinomas, but not Nonfunctioning Pituitary Adenomas, maintain a Functional Apoptotic RET/Pit1/ARF/p53 Pathway that is Blocked by excess GDNF. Endocrinology 2014; 155:4329-4340.
18 Petrossians P, Daly AF, Natchev E, Maione L, Blijdorp K, Sahnoun-Fathallah M, Auriemma R, Diallo AM, Hulting AL, Ferone D, Hana V Jr, Filipponi S, Sievers C, Nogueira C, Fajardo-Montañana C, Carvalho D, Hana V, Stalla GK, Jaffrain-Réa ML, Delemer B, Colao A, Brue T, Neggers SJCMM, Zacharieva S, Chanson P, Beckers A.. Acromegaly at diagnosis in 3173 patients from the Liege Acromegaly Survey (LAS) Database. Endocr Relat Cancer 2017; 24:505-518.
19 Jing S, Wen D, Yu Y, Holst PL, Luo Y, Fang M, Tamir R, Antonio L, Hu Z, Cupples R, Louis JC, Hu S, Altrock BW, Fox GM. GDNF-induced activation of the ret protein tyrosine kinase is mediated by GDNFR-alpha, a novel receptor for GDNF. Cell 1996; 85:1113-1124.
20 Treanor JJ, Goodman L, de Sauvage F, Stone DM, Poulsen KT, Beck CD, Gray C, Armanini MP, Pollock RA, Hefti F, Phillips HS, Goddard A, Moore MW, Buj-Bello A, Davies AM, Asai N, Takahashi M, Vandlen R, Henderson CE, Rosenthal A. Characterization of a multicomponent receptor for GDNF. Nature 1996; 382:80-83.
21 Baloh RH, Tansey MG, Golden JP, Creedon DJ, Heuckeroth RO, Keck CL, Zimonjic DB, Popescu NC, Johnson EM Jr, Milbrandt J. TrnR2, a novel receptor that mediates neurturin and GDNF signaling through Ret. Neuron 1997; 18:793-802.
22 Jing S, Yu Y, Fang M, Hu Z, Holst PL, Boone T, Delaney J, Schultz H, Zhou R, Fox GM. GFRalpha-2 and GFRalpha-3 are two new receptors for ligands of the GDNF family. J Biol Chem 1997; 272:33111-33117.
23 Baloh RH, Tansey MG, Lampe PA, Fahrner TJ, Enomoto H, Simburger KS Leitner ML, Araki T, Johnson EM Jr, Milbrandt J. Artemin, a Novel Member of the GDNF Ligand Family, Supports Peripheral and Central Neurons and Signals through the GFRα3-RET Receptor Complex. Neuron 1998; 21:1291-1302.
24 Milbrandt J, de Sauvage FJ, Fahrner TJ, Baloh RH, Leitner ML, Tansey MG, Lampe PA, Heuckeroth RO, Kotzbauer PT, Simburger KS, Golden JP, Davies JA, Vejsada R, Kato AC, Hynes M, Sherman D, Nishimura M, Wang LC, Vandlen R, Moffat B, Klein RD, Poulsen K, Gray C, Garces A, Henderson CE, Philips HS, Johnson EM Jr. Persephin, a novel neurotrophic factor related to GDNF and neurturin. Neuron 1998; 20:245-253.
25 Sidorova YA, Matlik K, Paveliev M, Lindahl M, Piranen E, Milbrandt J, Arumäe U, Saarma M, Bespalov MM Persephin signaling through GFRalpha1: the potential for the treatment of Parkinson's disease. Mol Cell Neurosci 2010; 44:223-232.
26 Olson LE, Tollkuhn J, Scafoglio C, Krones A, Zhang J, Ohgi KA, Wu W, Taketo MM, Kemler R, Grosschedl R, Rose D, Li X, Rosenfeld MG. Homeodomain-mediated beta-catenin-dependent switching events dictate cell-lineage determination. Cell 2006; 125:593-605.
27 Cushman LJ, Watkins-Chow DE, Brinkmeier ML, Raetzman LT, Radak AL, Lloyd RV, Camper SA. Persistent Prop1 expression delays gonadotrope differentiation and enhances pituitary tumor susceptibility. Hum Mol Genet 2001; 10:1141-1153.
28 Lindahl M, Timmusk T, Rossi J, Saarma M, Airaksinen MS. Expression and alternative splicing of mouse Gfra4 suggest roles in endocrine cell development. Mol Cell Neurosci 2000; 15:522-533.
29 Coya R, Alvarez CV, Perez F, Gianzo C, Dieguez C. Effects of TGF-beta1 on prolactin synthesis and secretion: an in-vitro study. J Neuroendocrinol 1999; 11:351-360.
30 Garcia A, Alvarez CV, Smith RG, Dieguez C. Regulation of Pit-1 expression by ghrelin and GHRP-6 through the GH secretagogue receptor. Mol Endocrinol 2001; 15:1484-1495.
31 Bravo SB, Garcia-Rendueles ME, Garcia-Rendueles AR, Rodrigues JS, Perez-Romero S, Garcia-Lavandeira M, Czarnocka B, Senra A, Lareu MV, Rodriguez-Garcia J, Cameselle-Teijeiro J, Alvarez CV. Humanized medium (h7H) allows long-term primary follicular thyroid cultures from human normal thyroid, benign neoplasm, and cancer. J Clin Endocrinol Metab 2013; 98:2431-2441.
32 Garcia-Rendueles A, Rodrigues JS, Garcia-Rendueles M, Suarez-Fariña M, Perez-Romero S, Barreiro F, Bernabeu I, Rodriguez-Garcia J, Fugazzola L, Sakai T, Liu F, Cameselle-Teijeiro J, Bravo SB, Alvarez CV. Rewiring of the apoptotic TGF-beta-SMAD/NFkappaB pathway through an oncogenic function of p27 in human papillary thyroid cancer. Oncogene 2017; 36:652-666.
33 Quelle DE, Zindy F, Ashmun RA, Sherr CJ. Alternative reading frames of the INK4a tumor suppressor gene encode two unrelated proteins capable of inducing cell cycle arrest. Cell 1995; 83:993-1000.
34 Duro D, Bernard O, Della Valle V, Berger R, Larsen CJ. A new type of p16INK4/MTS1 gene transcript expressed in B-cell malignancies. Oncogene 1995; 11:21-29.
35 Kotsinas A, Papanagnou P, Evangelou K, Trigas GC, Kostourou V, Townsend P, Gorgoulis VG. ARF: a versatile DNA damage response ally at the crossroads of development and tumorigenesis. Front Genet 2014; 5:236.
36 Valimaki N, Demir H, Pitkanen E, Kaasinen E, Karppinen A, Kivipelto L, Schalin-Jäntti C, Aaltonen LA, Karhu A. Whole-Genome Sequencing of Growth Hormone (GH)-Secreting Pituitary Adenomas. J Clin Endocrinol Metab 2015; 100:3918-3927.
37 Ronchi CL, Peverelli E, Herterich S, Weigand I, Mantovani G, Schwarzmayr T, Sbiera S, Allolio B, Honegger J, Appenzeller S, Lania AG, Reincke M, Calebiro D, Spada A, Buchfelder M, Flitsch J, Strom TM, Fassnacht M. Landscape of somatic mutations in sporadic GH-secreting pituitary adenomas. Eur J Endocrinol 2016; 174:363-372.
38 Bi WL, Horowitz P, Greenwald NF, Abedalthagafi M, Agarwalla PK, Gibson WJ, Mei Y, Schumacher SE, Ben-David U, Chevalier A, Carter S, Tiao G, Brastianos PK, Ligon AH, Ducar M, MacConaill L, Laws ER Jr, Santagata S, Beroukhim R, Dunn IF. Landscape of Genomic Alterations in Pituitary Adenomas. Clin Cancer Res 2017; 23: 1841-1851.
39 Bi WL, Greenwald NF, Ramkissoon SH, Abedalthagafi M, Coy SM, Ligon KL, Mei Y, MacConaill L, Ducar M, Min L, Santagata S, Kaiser UB, Beroukhim R, Laws ER Jr, Dunn IF. Clinical Identification of Oncogenic Drivers and Copy-Number Alterations in Pituitary Tumors. Endocrinology 2017; 158:2284-2291.
40 Tsai KY, MacPherson D, Rubinson DA, Nikitin AY, Bronson R, Mercer KL, Crowley D, Jacks T. ARF mutation accelerates pituitary tumor development in Rb+/- mice. Proc Natl Acad Sci U S A 2002: 99:16865-16870.
41 Chesnokova V, Zonis S, Rubinek T, Yu R, Ben-Shlomo A, Kovacs K, Wawrowsky K, Melmed S. Senescence mediates pituitary hypoplasia and restrains pituitary tumor growth. Cancer Res 2007; 67:10564-10572.
42 Chesnokova V, Zhou C, Ben-Shlomo A, Zonis S, Tani Y, Ren SG, Melmed S. Growth hormone is a cellular senescence target in pituitary and nonpituitary cells. Proc Natl Acad Sci U S A 2013; 110: E3331-3339.
43 Puig Domingo M. Treatment of acromegaly in the era of personalized and predictive medicine. Clin Endocrinol (Oxf) 2015; 83:3-14.
44 Kasuki L, Wildemberg LE, Gadelha MR. Management of Endocrine Disease: personalized medicine in the treatment of acromegaly. Eur J Endocrinol 2018; 178:R89-R100.
45 Taboada GF, Luque RM, Bastos W, Guimarães RF, Marcondes JB, Chimelli LM, Fontes R, Mata PJ, Filho PN, Carvalho DP, Kineman RD, Gadelha MR. Quantitative analysis of somatostatin receptor subtype (SSTR1-5) gene expression levels in somatotropinomas and non-functioning pituitary adenomas. Eur J Endocrinol 2007; 156:65-74.
46 Ferone D, de Herder WW, Pivonello R, Kros JM, van Koetsveld PM, de Jong T, Minuto F, Colao A, Lamberts SW, Hofland LJ. Correlation of in vitro and in vivo somatotropic adenoma responsiveness to somatostatin analogs and dopamine agonists with immunohistochemical evaluation of somatostatin and dopamine receptors and electron microscopy. J Clin Endocrinol Metab 2008; 93, 1412-1417.
47 Taboada GF, Luque RM, Neto LV, Machado Ede O, Sbaffi BC, Domingues RC, Marcondes JB, Chimelli LM, Fontes R, Niemeyer P, de Carvalho DP, Kineman RD, Gadelha MR. Quantitative analysis of somatostatin receptor subtypes (1-5) gene expression levels in somatotropinomas and correlation to in vivo hormonal and tumor volume responses to treatment with octreotide LAR. Eur J Endocrinol 2008; 158:295-303.
48 Neto LV, Machado Ede O, Luque RM, Taboada GF, Marcondes JB, Chimelli LM, Quintella LP, Niemeyer P Jr, de Carvalho DP, Kineman RD, Gadelha MR. Expression analysis of dopamine receptor subtypes in normal human pituitaries, nonfunctioning pituitary adenomas and somatotropinomas, and the association between dopamine and somatostatin receptors with clinical response to octreotide-LAR in acromegaly. J Clin Endocrinol Metab 2009; 94: 1931-1937.
49 Casar-Borota O, Heck A, Schulz S, Nesland JM, Ramm-Pettersen J, Lekva T, Alafuzoff I, Bollerslev J. Expression of SSTR2a, but not of SSTRs 1, 3, or 5 in somatotroph adenomas assessed by monoclonal antibodies was reduced by octreotide and correlated with the acute and long-term effects of octreotide. J Clin Endocrinol Metab 2013; 98:E1730-1739.
50 Gatto F, Feelders RA, van der Pas R, Kros JM, Waaijers M, Sprij-Mooij D, Neggers SJ, van der Lelij AJ, Minuto F, Lamberts SW, de Herder WW, Ferone D, Hofland LJ. Immunoreactivity score using an anti-sst2A receptor monoclonal antibody strongly predicts the biochemical response to adjuvant treatment with somatostatin analogs in acromegaly. J Clin Endocrinol Metab 2013; 98:E66-71.
51 Duran-Prado M, Saveanu A, Luque RM, Gahete MD, Gracia-Navarro F, Jaquet P, Dufour H, Malagón MM, Culler MD, Barlier A, Castaño JP. A potential inhibitory role for the new truncated variant of somatostatin receptor 5, sst5TMD4, in pituitary adenomas poorly responsive to somatostatin analogs. J Clin Endocrinol Metab 2010; 95: 2497-2502.
52 Venegas-Moreno E, Vazquez-Borrego MC, Dios E, Gros-Herguido N, Flores-Martinez A, Rivero-Cortés E, Japon MA, Luque RM, Castaño JP, Cano DA, Soto-Moreno A. Association between dopamine and somatostatin receptor expression and pharmacological response to somatostatin analogues in acromegaly. J Cell Mol Med 2018; 22:1640-1649.
53 Theodoropoulou M, Tichomirowa MA, Sievers C, Yassouridis A, Arzberger T, Hougrand O, Deprez M, Daly AF, Petrossians P, Pagotto U, Beckers A, Stalla GK. Tumor ZAC1 expression is associated with the response to somatostatin analog therapy in patients with acromegaly. Int J Cancer 2009; 125:2122-2126.
54 Fougner SL, Lekva T, Borota OC, Hald JK, Bollerslev J, Berg JP. The expression of E-cadherin in somatotroph pituitary adenomas is related to tumor size, invasiveness, and somatostatin analog response. J Clin Endocrinol Metab 2010; 95:2334-2342.
55 Gatto F, Biermasz NR, Feelders RA, Kros JM, Dogan F, van der Lely AJ, Neggers SJ, Lamberts SW, Pereira AM, Ferone D, Hofland LJ.L ow beta-arrestin expression correlates with the responsiveness to long-term somatostatin analog treatment in acromegaly. Eur J Endocrinol 2016; 174: 651-662.
56 Denes J, Kasuki L, Trivellin G, Colli LM, Takiya CM, Stiles CE, Barry S, de Castro M, Gadelha MR, Korbonits M. Regulation of aryl hydrocarbon receptor interacting protein (AIP) protein expression by MiR-34a in sporadic somatotropinomas. PLoS One 2015; 10: e0117107.
57 Kasuki L, Wildemberg LE, Neto LV, Marcondes J, Takiya CM, Gadelha MR. Ki-67 is a predictor of acromegaly control with octreotide LAR independent of SSTR2 status and relates to cytokeratin pattern. Eur J Endocrinol 2013; 169:217-223.
58 Ross FA, Hawley SA, Auciello FR, Gowans GJ, Atrih A, Lamont DJ, Hardie DG. Mechanisms of Paradoxical Activation of AMPK by the Kinase Inhibitors SU6656 and Sorafenib. Cell Chem Biol 2017; 24:813-824.e4.
59 Fumarola C, Caffarra C, La Monica S, Galetti M, Alfieri RR, Cavazzoni A, Galvani E, Generali D, Petronini PG, Bonelli MA. Effects of sorafenib on energy metabolism in breast cancer cells: role of AMPK-mTORC1 signaling. Breast Cancer Res Treat 2013; 141:67-78.
60 Prieto-Dominguez N, Ordonez R, Fernandez A, Garcia-Palomo A, Muntané J, Gonzalez-Gallego J, Mauriz JL. Modulation of Autophagy by Sorafenib: Effects on Treatment Response. Front Pharmacol 2016; 7:151.
61 Garcia-Lavandeira M, Quereda V, Flores I, Saez C, Diaz-Rodriguez E, Japon MA, Ryan AK, Blasco MA, Dieguez C, Malumbres M, Alvarez CV. A GRFa2/Prop1/stem (GPS) cell niche in the pituitary. PLoS One 2009; 4:e4815.
62 Sornson MW, Wu W, Dasen JS, Flynn SE, Norman DJ, O'Connell SM, Gukovsky I, Carrière C, Ryan AK, Miller AP, Zuo L, Gleiberman AS, Andersen B, Beamer WG, Rosenfeld MG. Pituitary lineage determination by the Prophet of Pit-1 homeodomain factor defective in Ames dwarfism. Nature 1996; 384:327-333.
63 Lindahl M, Poteryaev D, Yu L, Arumae U, Timmusk T, Bongarzone I, Aiello A, Pierotti MA, Airaksinen MS, Saarma M. Human glial cell line-derived neurotrophic factor receptor alpha 4 is the receptor for persephin and is predominantly expressed in normal and malignant thyroid medullary cells. J Biol Chem 2001; 276:9344-9351.

## Claims

1. An *in vitro* method for determining the prognosis of a patient suffering from acromegaly, comprising the steps of:
a. determining the expression level of ARF gene in a sample from said patient, and
b. comparing said expression level with a reference value,
wherein a decreased expression level of ARF gene compared to the reference value is indicative of bad prognosis, and
wherein an increased expression level of ARF gene compared to the reference value is indicative of a good prognosis.

2. The *in vitro* method according to claim 1, wherein a decreased expression level of ARF gene compared to the reference value is indicative of the presence of residual tumor after surgery and
wherein an increased expression level of ARF compared to the reference value is indicative of absence of residual tumor after surgery.

3. The *in vitro* method according to claim 1 or 2, wherein a decreased expression level of ARF gene compared to the reference value is indicative of resistance to a therapy with an analogue of somatostatin (SSA) and
wherein an increased expression level of ARF gene compared to the reference value is indicative of a good response to a therapy with an analogue of somatostatin (SSA).

4. The *in vitro* method according to claim 3, wherein the analogue of somatostatin is a first-generation analogue of somatostatin (fgSSA), preferably selected from octeotride or lanreotride.

5. The *in vitro* method according to any of claims 3 to 4, wherein the therapy with SSA is a first-line therapy following surgery.

6. The *in vitro* method according to any of claim 1 to 5, wherein the sample is a pituitary sample, preferably a surgical sample.

7. The *in vitro* method according to any of claims 1 to 6, wherein the determination of the expression level of the ARF gene is carried out by determining the levels of mRNA.

8. An *in vitro* method for predicting the clinical response of a patient suffering from acromegaly to a treatment with an analogue of somatostatin (SSA) comprising the steps of:
(a) determining the expression level of ARF gene in a sample from said patient and
(b) comparing said expression level with a reference value,
wherein a decreased expression level of ARF gene compared to the reference value is indicative of resistance to a therapy with an analogue of somatostatin (SSA) and
wherein an increased expression level of ARF compared to the reference value is indicative of a good response to a therapy with an analogue of somatostatin (SSA).

9. An *in vitro* method for selecting a patient suffering from acromegaly for a therapy with a SSA, comprising the steps of:
(a) determining the expression level of ARF gene in a sample from said patient and
(b) comparing said expression level with a reference value,
wherein the patient is selected for said therapy if the expression level of ARF gene is increased compared to the reference value.

10. An *in vitro* method for selecting a therapy for a patient suffering from acromegaly, comprising the steps of
(a) determining the expression level of ARF gene in a sample from said patient and
(b) comparing said expression level with a reference value,
wherein if the expression level of ARF gene is increased compared to the reference value, a therapy with a SSA is selected and
wherein if the expression level of ARF gene is decreased compared to the reference value, an alternative therapy is selected.

11. A Tyrosine Kinase Inhibitor (TKI) that blocks the RET/GDNF survival pathway for use in the treatment of acromegaly.

12. A Tyrosine Kinase Inhibitor (TKI) for use according to claim 11, wherein the TKI is Sorafenib.

13. Sorafenib for use according to claim 12, wherein the acromegaly is resistant to first-line therapy following surgery, preferably wherein the first-line therapy is a first-generation analogue of somatostatin (fgSSA), more preferably selected from octeotride or lanreotride.

14. Sorafenib for use according to claims 12 or 13, wherein it is administered in a dose below 800 mg/day, preferably below 600 mg per day, more preferably below 400 mg per day, and even more preferably below 200 mg/day.

15. Use of a reagent capable of determining the expression level of ARF gene in a sample from a subject suffering from acromegaly for determining the prognosis of said patient, or for predicting the clinical response of a patient suffering from acromegaly to a treatment with a SSA, or for selecting a patient suffering from acromegaly for a therapy with a SSA.
